# EUROPÄISCHE PATENTSCHRIFT

(11) **EP 2 385 933 B1**
(45) Veröffentlichungstag und Bekanntmachung des Hinweises auf die Patenterteilung: **25.06.2014**
(21) Anmeldenummer: 10700515.9
(22) Anmeldetag: 07.01.2010
(51) Int. Cl.: C07C 29/141, C07C 45/75, C07C 45/82

(54) **VERFAHREN ZUR HERSTELLUNG VON POLYMETHYLOLEN**
METHOD FOR PRODUCING POLYMETHYLOLS
PROCÉDÉ POUR LA PRÉPARATION DE POLYMÉTHYLOLS

(30) Priorität: 12.01.2009 EP 09150373
(43) Veröffentlichungstag der Anmeldung: 16.11.2011
(73) Patentinhaber: BASF SE, 67056 Ludwigshafen (DE)
(72) Erfinder: SCHLITTER, Stephan, Shanghai 200336 (CN); STEINIGER, Michael, 67435 Neustadt (DE); RITTINGER, Stefan, 68199 Mannheim (DE); SIRCH, Tilman, 67105 Schifferstadt (DE); MAAS, Steffen, 55270 Bubenheim (DE)
(86) Internationale Anmeldenummer: PCT/EP2010/050087
(87) Internationale Veröffentlichungsnummer: WO 2010/079187

(56) Entgegenhaltungen:
- WO-A1-98/28253
- WO-A1-2008/000650

## Beschreibung

Die Erfindung betrifft ein Verfahren zur Rückgewinnung von Komponenten aus einem Leichtsiedergemisch, das bei der Destillation von Hydrierausträgen aus der Herstellung von Polymethylolen anfällt.

Polymethylole, wie beispielsweise Neopentylglykol ("NPG") und Trimethyolpropan ("TMP"), werden im Kunststoffbereich zur Herstellung von Lacken, Beschichtungen, Polyurethanen und Polyestern eingesetzt.

Großtechnisch werden Polymethylole zumeist nach dem Cannizzaro-Verfahren hergestellt. Um Trimethylolpropan nach diesem Verfahren herzustellen, wird n-Butyraldehyd mit einem Überschuss Formaldehyd in Gegenwart einer anorganischen Base umgesetzt. Dabei entsteht zugleich ein Äquivalent eines anorganischen Formiats als Koppelprodukt. Die Trennung des Salzes von Trimethylolpropan ist kompliziert und erfordert zusätzlichen Aufwand. Außerdem muss das anorganische Salz - falls es nutzbringend verwertet werden soll - aufgearbeitet und gereinigt werden. Der Anfall des Koppelproduktes stellt ansonsten einen Verlust der stöchiometrisch eingesetzten Mengen an Natronlauge und Formaldehyd dar. Zusätzlich sind die Ausbeuten bei dieser anorganischen Cannizzaro-Reaktion in Bezug auf n-Butyraldehyd unbefriedigend, da im Verlauf der Reaktion hochsiedende Bestandteile gebildet werden, die nicht weiterverwertet werden können.
Ähnliche Probleme, wie für Trimethylolpropan geschildert, liegen bei der Herstellung von anderen Polymethylolen wie Trimethylolethan (aus n-Propanal und Formaldehyd) oder Trimethylolbutan (aus n-Pentanal und Formaldehyd) oder Neopentylglykol (aus Isobutyraldehyd und Formaldehyd) vor.

Zur Vermeidung dieser Nachteile wurde durch die WO 98/28253 ein mehrstufigen Verfahren zur Herstellung von Polymethylolen bekannt, bei dem Aldehyde mit 2 bis 24 C-Atomen in einer ersten Stufe (Aldolreaktion) mit Formaldehyd unter Verwendung von tertiären Aminen als Katalysator zunächst zu den entsprechenden Methylolalkanalen kondensiert und anschließend in einer weiteren Stufe (Hydrierung) zu den entsprechenden Polymethylolen hydriert werden. Dieses mehrstufige Verfahren wird üblicherweise als Hydrierverfahren bezeichnet. Dieses Verfahren ist koppelproduktarm.

Nach der ersten Stufe des Hydrierverfahrens werden nicht umgesetzte Aldehyde und ein Teil der Amin-Base im Allgemeinen von den gebildeten Methylolalkanalen destillativ abgetrennt und zurückgeführt.
Im Destillationssumpf verbleiben - neben den gebildeten Methylolalkanalen - Wasser, die Addukte aus Ameisensäure und den eingesetzten tertiären Aminen (Amin-Formiat) und Ameisensäure selbst.

In der Regel wird das Polymethylolalkanal nach diesen Verfahren als 20 bis 70 Gew.-%ige wässrige Lösung erhalten.
Die Polymethylolalkanal-haltige Lösung wird in einer zweiten Stufe hydriert, um die Polymethyolalkanale zu den entsprechenden Polymethylolen, wie TMP oder NPG, umzusetzen.
Der Reaktionsaustrag aus der Hydrierung ist üblicherweise ein wässriges Polymethylolgemisch, welches Polymethylol, tertiäres Amin, Wasser sowie organische Nebenkomponenten, wie beispielsweise ein Addukt aus tertiärem Amin und Ameisensäure (Amin-Formiat) enthält.
Das wässrige Polymethylolgemisch wird deshalb üblicherweise aufgereinigt, in dem Leichtsieder von der Polymethylolverbindung destillativ abgetrennt werden.

Bei der destillativen Abtrennung der Leichtsieder fällt im Kondensator als Kopfprodukt ein Gemisch von Leichtsiedern an. Beispielsweise kann das Leichtsiedergemisch tertiäres Amin, nicht umgesetztes Aldehyd und Wasser enthalten. Insbesondere enthält dass Leichtsiedergemisch Alkohole, die durch Hydrierung der in das Verfahren eingesetzten Alkanale entstanden sind, wie iso-Butanol aus Isobutyraldehyd oder n-Butanol aus n-Butyraldehyd, sowie Methanol aus Formaldehyd.

Die Rückführung eines solchen Leichtsiedergemisches in die erste Stufe des Hydrierverfahrens (Aldolreaktion) ist nicht vorteilhaft, da das Leichtsiedergemisch Methanol und den entsprechenden Alkohol aus dem nicht umgesetzten Aldehyd (im NPG-Prozess iso-Butanol) enthält, welche einen negativen Effekt in der Aldolreaktion ausüben.
Höhere Methanolgehalte führen beispielsweise zu Nebenprodukten, wie Neopentylglykolmethylether und/oder Methylacetalen durch Reaktion von nichtumgesetzten Aldehyden, wie Isobutyraldehyd und Formaldehyd bzw. Methylolalkanen mit Methanol. Weitere Nebenprodukte sind beispielsweise 3,3-Dimethoxy-2,2-dimethylpropanol, welches bei der Trimethylolpropan bzw. Neopentylglykolherstellung anfällt.
Die Bildung der genannten Nebenprodukte führt zu einer Verringerung der Ausbeute in Bezug auf den als Edukt eingesetzten Aldehyd.
Methanol führt in der Aldolreaktion nicht nur zu einer Verringerung der Ausbeute durch Nebenreaktionen, es ist zudem nur sehr schwer von den übrigen Komponenten abzutrennen, da Methanol und die eingesetzten Aldehyde einen ähnlichen Siedepunkt aufweisen. Will man jedoch die nicht umgesetzten Ausgangsaldehyde in die Aldolreaktion zurückführen, so bedeutet dies, dass auch eine signifikante Menge der Aldehyde zusammen mit Methanol abgetrennt werden muss, damit Aldehyd mit einem möglichst geringen Methanolgehalt in den Prozess zurückgeführt werden kann. Ansonsten würde es zu einer Aufpegelung von Methanol in der Aldolreaktion kommen, was die voranstehend beschriebene verstärkte Bildung von Nebenprodukten zur Folge hätte.

Ähnlich führt das Vorhandensein von iso-Butanol in der Aldolreaktion zu einer verschlechterten Ausbeute, da es wasserdampfflüchtig ist und nur schwer von dem als Edukt eingesetzten Aldehyd destillativ abgetrennt werden kann.

Eine Verwertung des Leichtsiedergemisches durch destillative Trennung ist nicht trivial, da die Siedepunkte der im Leichtsiedergemisch enthaltenen Substanzen eng beisammen liegen und diese Substanzen zum Teil Azeotrope bilden.
Nachteilig ist insbesondere das Vorhandensein von iso-Butanol bei der NPG-Herstellung, da iso-Butanol mit Wasser ein leichtsiedendes Heteroazeotrop mit Mischungslücke bildet.
Weiterhin sind in dem Leichtsiedergemisch gelöster Kohlendioxid, der in der Hydrierstufe durch Hydrierung von Formaldehyd entsteht, und Ameisensäure vorhanden, die nicht frei sondern als Salze mit dem Amin in der wässrigen Lösung vorliegt. Durch das Vorhandensein von Salzen wird die Destillation weiter erschwert.

Im Rahmen dieser Erfindung wurde nun gefunden, dass selbst durch eine mehrstufige Destillation des Leichtsiedergemisches aus Stufe d) eine Auftrennung in verwertbare Komponenten dadurch erschwert wird, dass die im Leichtsiedergemisch vorhandenen, nicht umgesetzten Aldehyde die Flüchtigkeit der eingesetzten tertiären Amine, insbesondere von TMA, reduzieren, so dass eine Abtrennung der tertiären Amine von den anderen organischen Bestandteilen nur unzureichend gelingt oder mit hohen Ausbeuteverlusten verbunden ist.

Aufgabe der vorliegenden Erfindung war es ein Verfahren zur Aufreinigung eines Leichtsiedergemisches, das bei der Herstellung von Polymethylole anfällt, zu entwickeln, dass es ermöglicht, die in diesem Gemisch vorhandenen Wertstoffe zu nutzen. Insbesondere sollte das als basischer Katalysator eingesetzte tertiäre Amin zurückgewonnen werden, um es in die Aldolreaktion zurückführen zu können. Hierbei sollte das zurückgeführte tertiäre Amin weitestgehend frei von Methanol sein, so dass es nicht zu einer Erhöhung oder Aufpegelung der Methanol-Konzentration in der Aldolreaktion kommt, was die verstärkte Bildung von Nebenprodukten zur Folge hätte. Insbesondere sollte auch eine nahezu vollständige Abtrennung der eingesetzten tertiären Amine erreicht werden, wobei die Ausbeuteverluste minimiert werden sollten. Weiterhin war es ein Ziel, den apparatetechnischen Aufwand für die Destillation so gering wie möglich zu halten, um ein wirtschaftliches Aufarbeitungsverfahren zur Verfügung stellen zu können.
Die Aufgabe der vorliegenden Erfindung wurde gelöst durch ein

Verfahren zur Rückgewinnung von Komponenten aus einem Leichtsiedergemisch, das bei der Destillation von Hydrierausträgen aus der Herstellung von Polymethylolen anfällt, durch mehrstufige Destillation des Leichtsiederaustrags, enthaltend ein tertiäres Amin, Wasser, Methanol, Polymethylol der Formel (I), 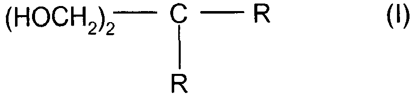 Methylolalkanal der Formel (II), 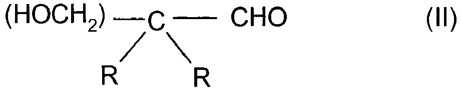 Alkohol der Formel (III)), 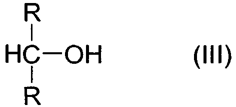 und ein Alkanal mit einer Methylengruppe in α-Stellung zur Carbonylgruppe,
und in der R jeweils unabhängig voneinander eine weitere Methylolgruppe oder eine Alkylgruppe mit 1 bis 22 C-Atomen oder eine Aryl- oder Aralkylgruppe mit 6 bis 22 C-Atomen bedeutet,
wobei in einer ersten Destillationsstufe eine Auftrennung in eine höhersiedende, überwiegend wasserreiche Fraktion und in eine das tertiäre Amin enthaltende leichtersiedende wässrige, organische Fraktion erfolgt,
und in der zweiten Destillationsstufe die wässrige, organische Fraktion aus der ersten Destillationsstufe in eine überwiegend aminhaltige Fraktion und eine weitere aminabgereicherte Fraktion aufgetrennt wird,
dadurch gekennzeichnet, dass das tertiäre Amin Trimethylamin oder Triethylamin ist und die Sumpftemperatur in der zweiten Destillationsstufe 110°C und mehr beträgt.

Das in das erfindungsgemäße Verfahren eingesetzte Leichtsiedergemisch wird bevorzugt in einer mehrstufigen Reaktion erhalten, wobei man in Stufe a) Alkanale in einer Aldolreaktion mit Formaldehyd in Gegenwart von tertiären Aminen als Katalysator zu Methylolalkanalen der Formel (II) kondensiert, und man anschließend in Stufe b) das aus Stufe a) erhaltenen Reaktionsgemisches in einen Sumpf, der überwiegend Verbindungen der Formel (II) enthält, und einen Kopfstrom, der Leichtsieder enthält, destillativ auftrennt, und man in Stufe c) den Sumpfaustrag aus Stufe b) hydriert und man anschließend in einer Stufe d) den Austrag aus Stufe c) destilliert, wobei das Leichtsiedergemisch aus Stufe d) abgetrennt wird.

In der ersten Verfahrensstufe a) (Aldolreaktion) werden im Allgemeinen Alkanale in einer Aldolreaktion mit Formaldehyd in Gegenwart von tertiären Aminen als Katalysator umgesetzt.

Formaldehyd wird in der Regel als wässrige Formaldehydlösung in das Verfahren eingesetzt. Technisch verfügbarer Formaldehyd wird üblicherweise in wässriger Lösung in Konzentrationen von 30, 37 und 49 Gew.-% vertrieben. Es ist jedoch auch möglich Formaldehydlösungen von bis zu 60 Gew.-% in das Verfahren einzusetzen.

Technisches Formaldehyd enthält in der Regel herstellungsbedingt Ameisensäure. Die Abbauprodukte von Ameisensäure können die Standzeit des Hydrierungskatalysators in der nachfolgenden Hydrierstufe verringern, was eine Verringerung der Ausbeute an Polymethylolen zur Folge haben kann. In einer besonderen Ausführungsform wird Formaldehyd eingesetzt, welcher einen Ameisensäuregehalt von 150 ppm oder weniger aufweist. Ein solcher Formaldehyd kann, wie in der Anmeldung PCT/EP2008/052240 beschrieben, durch Behandlung von Formaldehyd bzw. einer wässrigen Formaldehydlösung mit basischen Ionentauschern erhalten werden.

In die Aldolreaktion (Stufe a)) können Alkanale mit einer Methylengruppe in α-Stellung zur Carbonylgruppe eingesetzt werden.
So können bevorzugt aliphatische Alkanale mit 2 bis 24 C-Atomen als Ausgangsmaterialien verwendet werden, die geradkettig oder verzweigt sein oder auch alicyclische Gruppen enthalten können.
Ebenso können araliphatische Alkanale als Ausgangsstoffe eingesetzt werden, vorausgesetzt, dass sie eine Methylengruppe in α-Stellung zur Carbonylgruppe enthalten. Im allgemeinen werden Aralkylalkanale mit 8 bis 24 C-Atomen, vorzugsweise mit 8 bis 12 C-Atomen als Ausgangsmaterialien verwendet, beispielsweise Phenylacetaldehyd. Besonders bevorzugt werden aliphatische Alkanale mit 2 bis 12 C-Atomen, beispielsweise 3-Ethyl-, 3-n-Propyl-, 3-Isopropyl-, 3-n-Butyl-, 3-Isobutyl-, 3-sek.-Butyl-, 3-tert.-Butyl-butanal sowie entsprechende -n-pentanale, -n-hexanale, -n-heptanale; 4-Ethyl-, 4-n-Propyl-, 4-Isopropyl-, 4-n-Butyl-, 4-Isobutyl-, 4-sek.-Butyl-, 4-tert.-Butyl-pentanale, - n-hexanale, -n-heptanale; 5-Ethyl-, 5-n-Propyl-, 5-Isopropyl-, 5-n-Butyl-, 5-Isobutyl-, 5-sek.-Butyl-, 5-tert.-Butyl-n-hexanale, -n-heptanale; 3-Methyl-hexanal, 3-Methylheptanal; 4-Methylpentanal, 4-Methylheptanal, 5-Methyl-hexanal, 5-Methylheptanal; 3,3,5-Trimethyl-n-pentyl-, 3,3-Diethylpentyl-, 4,4-Diethylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl, 3,4-Dimethylheptyl-, 3,5-Dimethylheptyl-, 4,4-Dimethylheptyl-, 3,3-Diethylhexyl-, 4,4-Dimethylhexyl-, 4,5-Dimethylhexyl-, 3,4-Dimethylhexyl-, 3,5-Dimethylhexyl-, 3,3-Dimethylhexyl-, 3,4-Diethylhexyl-, 3-Methyl-4-ethylpentyl, 3-Methyl-4-ethylhexyl-, 3,3,4-Trimethylpentyl-, 3,4,4-Trimethylpentyl-, 3,3,4-Trimethylhexyl-, 3,4,4-Trimethylhexyl-, 3,3,4,4-Tetramethylpentylaldehyd; insbesondere C₂- bis C₁₂-n-Alkanale.
Neben dem besonders bevorzugten Einsatz von Isobutyraldehyd, das zur Herstellung von Neopentylglykol eingesetzt wird, können vorzugsweise außerdem n-Butyraldehyd zur Herstellung von Trimethylolpropan, Acetaldehyd zur Herstellung von Pentaerythrit, Propionaldehyd zur Herstellung von Trimethylolethan und n-Pentanal zur Herstellung von Trimethylolbutan als Ausgangsverbindungen eingesetzt werden.

Isobutyraldehyd wird in der Regel in reiner Form (GC FI% > 99%) in das Verfahren eingesetzt. Kommerziell verfügbarer Isobutyaraldehyd wird üblicherweise in Reinheiten 99,5% (ohne Wasser) mit einem n-Butyraldehydgehalt von weniger als 0,1 % angeboten, wobei der Wasseranteil in der Regel bis zu 2,5% beträgt.
Ein hoher Isobutyraldehydgehalt ist in der Regel vorteilhaft, da n-Butyraldehyd zu vermehrter TMP-Bildung führt, welches von NPG abgetrennt werden muss. Zudem können weitere Nebenprodukte gebildet werden, wie Isobutanol oder Isobuttersäure, die meist nach der Hydrierung als leichtsiedenede Alkohole abgetrennt werden müssen. Es kann aber auch Isobutyraldehyd mit einem Gehalt von weniger als 99,5% eingesetzt werden, beispielsweise mit einem Gehalt von 95 bis 98%.

Als tertiäre Amine werden Trimethylamin ("TMA") oder Triethylamin ("TEA") eingesetzt. Besonders bevorzugt wird Trimethylamin ("TMA") als tertiäres Amin in die Reaktion eingesetzt.

Die Aldolreaktion kann mit oder ohne Zusatz von organischen Lösungsmitteln oder Lösungsvermittlern durchgeführt werden. Der Zusatz von Lösungsmitteln oder Lösungsvermittlern kann sich insbesondere bei der Verwendung langkettiger Alkanale als Ausgangsmaterialien als vorteilhaft erweisen. Durch den Einsatz von Lösungsmitteln, die geeignete niedrigsiedende azeotrope Gemische mit den leichtsiedenden Verbindungen bei den einzelnen Destillationen des erfindungsgemäßen Verfahrens bilden, kann gegebenenfalls der Energieaufwand bei diesen Destillationen erniedrigt und/oder die destillative Abtrennung der Leichtsieder von den hochsiedenden Verbindungen erleichtert werden.
Als Lösungsmittel sind z. B. cyclische und acyclische Ether, wie THF, Dioxan, Methyltert.butylether oder Alkohole, wie Methanol, Ethanol oder 2-Ethylhexanol geeignet.

Bei der Aldolreaktion beträgt das Molverhältnis von jeweils frisch zugesetztem Alkanal zu der hinzugefügten Menge Formaldehyd zweckmäßigerweise zwischen 1:1 und 1:5, bevorzugt 1:1 bis 1:3.

Die Menge an bei der Aldolreaktion zugesetztem tertiärem Aminkatalysator beträgt in Bezug auf das zugesetzte Alkanal in der Regel 0,001 bis 0,2, bevorzugt 0,01 bis 0,07 Äquivalente, d. h. das Amin wird üblicherweise in katalytischen Mengen eingesetzt.

Die Aldolreaktion wird im allgemeinen bei einer Temperatur von 5 bis 100°C, bevorzugt von 15 bis 80°C durchgeführt und die Verweilzeit wird in Abhängigkeit von der Temperatur im allgemeinen auf 0,25 bis 12 Stunden eingestellt.

Die für die Aldolreaktion beschriebenen Reaktionsführungen können bei einem Druck von im allgemeinen 1 bis 30 bar, vorzugsweise 1 bis 15 bar, besonders bevorzugt 1 bis 5 bar, zweckmäßigerweise unter dem Eigendruck des betreffenden Reaktionssystems, durchgeführt werden.

Die Aldolreaktion kann diskontinuierlich oder kontinuierlich durchgeführt werden. Die Aldolreaktion kann in einem Rührkesselreaktor oder in einem Reaktionsrohr durchgeführt werden. Vorzugsweise wird die Aldolreaktion in einem kontinuierlich betriebenen Rührkesselreaktor oder einer kontinuierlich betriebenen Rührkesselkaskade durchgeführt. Zur Einstellung der Verweilzeit kann ein Teil des Reaktionaustrags aus einem Rührkessel in den jeweiligen Rührkesselreaktor zurückgeführt werden.

Der Austrag aus der Aldolreaktion enthält üblicherweise nicht umgesetzte Ausgangsverbindungen, wie Formaldehyd, Alkanale, sowie den eingesetzten tertiären Aminkatalysator und gegebenenfalls Wasser.

Weiterhin enthält der Austrag aus der Aldolreaktion ein Methylolalkanal der Formel (II), 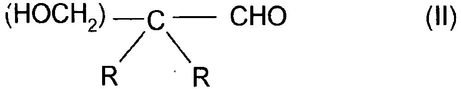 in der R jeweils unabhängig voneinander eine weitere Methylolgruppe oder eine Alkylgruppe mit 1 bis 22 C-Atomen oder eine Aryl- oder Aralkylgruppe mit 6 bis 22 C-Atomen bedeutet. Beispiele für Methylolalkanale sind Hydroxypivalinaldehyd, das bei der Verwendung von Isobutyraldehyd als Edukt gebildet wird oder Dimethylolbutanol, das bei der Verwendung von n-Butyraldehyd als Edukt gebildet wird.
Üblicherweise enthält der Austrag auch Verunreinigungen und Nebenprodukte aus der Aldolreaktion, wie Ameisensäure, die durch Cannizzaro- oder Tischtschenko-Reaktion aus Formaldehyd entstehen können, und Formiat-Salze der eingesetzten Aminkatalysatoren, wie Trimethylammoniumformiat.

Der Austrag aus der Aldolreaktion wird anschließend üblicherweise destillativ aufgetrennt (Stufe b)).
Hierbei wird der Austrag aus der Aldolreaktion einer Destillationsvorrichtung, üblicherweise eine Kolonne, zugeführt, in der es in leichter und schwerer flüchtige Bestandteile aufgetrennt wird.
Dabei werden die Destillationsbedingungen in der Regel so gewählt, dass sich eine Fraktion aus Leichtsiedern bildet, in der als wesentliche Komponenten nicht umgesetztes Alkanal und gegebenenfalls Wasser, Formaldehyd und Methanol enthalten sind.
Diese sogenannte Leichtsiederfraktion kann in die erste Stufe des Hydrierverfahrens, der Aldolreaktion, zurückgeführt werden oder einer weiteren Aufarbeitungsstufe zugeführt werden.

Nach Abtrennung der Leichtsiederfraktion verbleibt bei der geschilderten destillativen Aufarbeitung ein schwerer flüchtiges Sumpfprodukt, das im Wesentlichen aus Methylolalkanal (II), beispielsweise Hydroxypivalinaldehyd, Wasser, Ameisensäure sowie Amin-Formiat besteht.

Bei der Verwendung von TMA als tertiäres Amin, werden die Destillationsbedingungen so gewählt, dass TMA auch zum Teil in der in Leichtsiederfraktion enthalten ist und zum geringen Teil im Sumpfprodukt vorhanden ist. Bei der Verwendung von TEA, werden die Destillationsbedingungen so gewählt, dass TEA im Sumpfprodukt angereichert werden.

Die destillative Abtrennung sollte bevorzugt bei moderatem Druck erfolgen, um nicht durch erhöhte Temperatur die Methylolalkanale (II) zu zersetzen. Beispielsweise kann sich Hydroxypivalinaldehyd in Hydroxypivalinsäure-Neopentylglykolester (HPN) umsetzen. Andererseits sollte der Druck nicht zu gering sein, um noch am Kopf die Leichtsieder Alkanal, wie Isobutyraldehyd, und ggf. Amin-Base, beispielsweise Trimethylamin, zu kondensieren.
Die Destillation sollte auch deshalb nicht bei zu niedrigem Druck stattfinden, da in der Regel unterhalb von etwa 60°C die Löslichkeit von Alkanal (II), wie Hydroxypivalinaldehyd (HPA), in der wässrigen Lösung auf etwa 3 Gew.-% abfällt, in Abhängigkeit vom Alkanal- und Methanol-Gehalt.
Weiterhin sollte die Auftrennung des Austrags aus der Adolreaktion derart erfolgen, dass die Methanol-Menge im Leichtsiederstrom so gering wie möglich gehalten wird, damit sich die Methanolkonzentration in der Aldolreaktion nicht aufpegelt. Methanol wird in der Regel über die wässrige Formaldehyd-Lösung eingeschleppt, die je nach Herstellungsbedingungen etwa 0,5 bis 3 Gew.-% Methanol enthält.
Der Siedepunkt von Methanol ist in der Regel niedriger als der des nicht umgesetzten Alkanals, so dass sich Methanol am Kolonnenkopf anreichert und es somit zu einer Aufpegelung der Methanolkonzentration im Prozess kommt.
Um die Methanolkonzentration niedrig zu halten können verschiedene Maßnahmen getroffen werden.
Zum einen ist es vorteilhaft methanolarmes Formaldehyd als Edukt in die Aldolreaktion einzusetzen.
Weiterhin ist es möglich Methanol zusammen mit nicht umgesetzten Alkanal aus dem Prozess auszuschleusen, was zu einem Verlust an Alkanal führt.
In einer bevorzugten Ausführungsform wird die Destillation jedoch unter spezifischen Bedingungen durchgeführt, so dass Methanol ausreichend im Kolonnensumpf zurückgehalten wird. Diese bevorzugte Ausführungsform der destillativen Auftrennung des Austrags aus der Aldolreaktion ist in der Anmeldung PCT/EP2008/052240 beschrieben.
In dieser Ausführungsform wird die destillative Auftrennung in eine Leichtsiederfraktion und das Sumpfprodukt im allgemeinen bei 50 bis 200°C, vorzugsweise bei 90 bis 160°C und bei einem Druck von im allgemeinen 0,1 mbar bis 10 bar, vorzugsweise von 0,5 bis 5 bar, insbesondere bei Atmosphärendruck, in einer Destillationskolonne durchgeführt. Die Destillationskolonne wird üblicherweise bei einem Kopfdruck im Bereich von 0,5 bis 1,5 bar betrieben.
Im Kopfbereich ist bevorzugt eine zweistufige Kondensation vorgesehen, bei der die Brüden zunächst in einen bei einer Temperatur im Bereich von 50 bis 80°C betriebenen Partialkondensator geführt werden, dessen Kondensat zumindest teilweise in die Destillationskolonne zurückgeführt wird, und bei der die im Partialkondensator nicht kondensierten Brüden einem nachgeschalteten, bei einer Temperatur im Bereich von - 40 bis +30°C betriebenen Kondensator zugeführt werden, dessen Kondensat zumindest teilweise ausgeschleust wird.
Vorzugsweise wird das Kondensat des Partialkondensators zu mehr als 70 Gew.-%, besonders bevorzugt vollständig in die Destillationskolonne zurückgeführt. Dabei wird das Kondensat vorzugsweise in den Kolonnenkopf zurückgeführt. Das Kondensat des nachgeschalteten Kondensators wird vorzugsweise zu mindestens 70 Gew.-%, ausgeschleust.
Der Partialkondensator wird bei einer Temperatur im Bereich von 50 bis 80°C, vorzugsweise 55 bis 60°C betrieben. Der nachgeschaltete Kondensator wird bei einer Temperatur im Bereich von -40 bis +30°C, vorzugsweise -10 bis +10°C betrieben. Der Kopfdruck beträgt besonders bevorzugt 1 bis 1,2 bar.
Der Sumpf der Destillationskolonne ist bevorzugt mit einem Verdampfer mit kurzer Verweilzeit verbunden, der bei einer Temperatur im Bereich von 90 bis 130°C, besonders bevorzugt von 100 bis 105°C betrieben wird. Dabei handelt es sich bei dem Verdampfer besonders bevorzugt um einen Fallfilmverdampfer, ferner können bevorzugt ein Wischfilmverdampfer oder ein Kurzwegverdampfer eingesetzt werden. Wesentlich ist dabei, dass eine kurze Verweilzeit und damit eine geringe thermische Belastung erreicht wird. Der Verdampfer kann in geeigneter Weise mit Wärme versorgt werden, beispielsweise mit 4 bar Dampf.
Bevorzugt weist die Destillationskolonne Einbauten zur Erhöhung der Trennleistung auf. Dabei wird der Reaktionsaustrag der Aldolisierung vorzugsweise in einem räumlichen Bereich zwischen ¼ und ¾ der theoretischen Böden der Destillationskolonne zugeführt, besonders bevorzugt in einem räumlichen Bereich zwischen 1/3 und 2/3 der theoretischen Böden der Destillationskolonne. Beispielsweise kann die Zuführung etwas oberhalb der Mitte der theoretischen Böden erfolgen (Verhältnis 3:4).
Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Holdup im Vergleich zu beispielsweise Ventilböden.
Im Partialkondensator fällt als Kondensat überwiegend Wasser an, das vorzugsweise der Kolonne vollständig als Rücklauf zugeführt wird. Bei der Herstellung von NPG kann beispielsweise ein Gemisch als Kondensat erhalten werden, das etwa 10 Gew.-% Isobutyraldehyd, etwa 5 Gew.-% Amin-Base wie Trimethylamin, etwa 1 Gew.-% Hydroxypivalinaldehyd und etwa 5 Gew.-% Methanol neben Wasser enthält, wenn Isobutyraldehyd als Edukt verwendet wird. In diesem Falle enthalten die Restbrüden die überwiegende Menge an Isobutyraldehyd und Amin-Base wie Trimethylamin. Diese werden in dem nachgeschalteten Kondensator möglichst vollständig niedergeschlagen. Als Kühlmedium kann hierbei bevorzugt möglichst kaltes Wasser (z. B. etwa 5°C) oder eine Kältemischung (z. B. Glykol-Wasser mit beispielsweise -20°C) verwendet werden. Vorzugsweise wird ein mit Methylolalkanal (II), beispielsweise Hydroxypivalinaldehyd oder Dimethylolbutanal, angereichertes Gemisch aus dem Sumpf des Verdampfers ausgeschleust. Auch ein Ausschleusen aus dem Umlauf ist möglich.

Das schwerer flüchtiges Sumpfprodukt aus der destillativen Auftrennung des Austrags aus der Aldolreaktion kann zur Verminderung der thermischen Belastung vor der weiteren Aufarbeitung in einem Kühler mit einer Kühlertemperatur im Bereich von 50 bis 80°C, besonders bevorzugt 55 bis 60°C, abgekühlt werden.

Der so erhaltene Sumpfaustrag aus Stufe b) kann nachfolgend in Stufe c) hydriert werden.

Der Sumpfaustrag aus der Stufe b) des Hydrierverfahrens enthält Methylolalkanal der allgemeinen Formel (II) und wird in Stufe c) des Hydrierverfahrens zu den entsprechenden Polymethylolen hydriert ("Hydrierung").

In der Hydrierung werden vorzugsweise Katalysatoren eingesetzt, die mindestens ein Metall der Nebengruppe 8 bis 12 des Periodensystems der Elemente wie Fe, Ru, Os, Co, Rh, Ir, Ni, Pd, Pt, Cu, Ag, An, Zn, Cd, Hg, bevorzugt Fe, Co, Ni, Cu, Ru, Pd, Pt, besonders bevorzugt Cu, bevorzugt auf einem Trägermaterial enthalten.
Als Trägermaterial wird vorzugsweise ein Trägermaterial aus den Oxiden des Titans, Zirkoniums, Hafniums, Siliziums und/oder Aluminiums verwendet.
Die Herstellung der verwendbaren Katalysatoren kann nach aus dem Stand der Technik bekannten Verfahren zur Herstellung derartigen Trägerkatalysatoren erfolgen. Bevorzugt verwendet werden können auch Trägerkatalysatoren, die Kupfer auf einem Aluminiumoxid- oder Titandioxid-haltigen Trägermaterial in An- oder Abwesenheit eines oder mehrer der Elemente Magnesium, Barium, Zink oder Chrom aufweist. Derartige Katalysatoren und ihre Herstellung sind aus WO 99/44974 bekannt.

Weiterhin sind kupferhaltige Trägerkatalysatoren wie sie z.B. in WO 95/32171 beschrieben sind und die in EP-A 44 444 und DE 19 57 591 offenbarten Katalysatoren für die Hydrierung geeignet.

Die Hydrierung kann diskontinuierlich oder kontinuierlich z.B. in einem mit einer Katalysatorschüttung gefüllten Reaktorrohr durchgeführt werden, bei dem die Reaktionslösung über die Katalysatorschüttung z.B. in Riesel- oder Sumpffahrweise, wie in DE-A 19 41 633 oder DE-A 20 40 501 beschrieben, geleitet wird. Es kann vorteilhaft sein, einen Teilstrom des Reaktionsaustrages, gegebenenfalls unter Kühlung, zurückzuführen und wieder über das Katalysatorfestbett zu leiten. Ebenso kann es vorteilhaft sein, die Hydrierung in mehreren hintereinander geschalteten Reaktoren durchzuführen, beispielsweise in 2 bis 4 Reaktoren, wobei in den einzelnen Reaktoren vor dem letzten Reaktor, die Hydrierreaktion nur bis zu einem Teilumsatz von z.B. 50 bis 98 % durchgeführt wird und erst im letzten Reaktor die Hydrierung vervollständigt wird. Dabei kann es zweckmäßig sein, den Hydrieraustrag aus dem vorangehenden Reaktor vor dessen Eintritt in den nachfolgenden Reaktor zu kühlen, beispielsweise mittels Kühlvorrichtungen oder durch Eindüsen kalter Gase, wie Wasserstoff oder Stickstoff oder Einleiten eines Teilstroms an kalter Reaktionslösung.

Die Hydriertemperatur liegt im Allgemeinen zwischen 50 und 180°C, bevorzugt 90 und 140°C. Als Hydrierdruck werden im allgemeinen 10 bis 250 bar, bevorzugt 20 bis 120 bar angewandt.

Der Hydrierfeed wird in der Regel vor dem Hydrierreaktoreingang mit tertiärem Amin vermischt bis der Hydrieraustrag einen pH-Wert von 7 bis 9 aufweist. Es ist auch möglich, den Hydrierfeed und das tertiäre Amin getrennt in den Reaktor einzuspeisen und dort zu vermischen. Als tertiäre Amine können die zuvor genannten tertiären Amine, insbesondere TMA, eingesetzt werden.

Der Reaktionsaustrag aus der Hydrierung (Stufe c)) ist üblicherweise ein wässriges Polymethylolgemisch, welches ein Polymethylol der Formel (I), 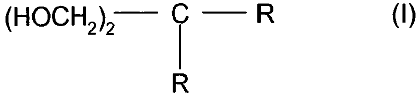 in der R jeweils unabhängig voneinander eine weitere Methylolgruppe oder eine Alkylgruppe mit 1 bis 22 C-Atomen oder eine Aryl- oder Aralkylgruppe mit 6 bis 22 C-Atomen bedeutet, ein tertiäres Amin, Wasser sowie das Addukt aus tertiärem Amin und Ameisensäure (Amin-Formiat) enthält.

Das wässrige Polymethylolgemisch weist bevorzugt folgende Zusammensetzung auf:
20 bis 90 Gew.-% Polymethylol (I),
0 bis 10 Gew.-% Methanol,
0 bis 5 Gew.-% tert. Amin,
0 bis 5 Gew.% organische Nebenverbindungen,
0,01 bis 5 Gew.% des Addukts aus tertiärem Amins und Ameisensäure (Amin-Formiat),
Rest Wasser.

Besonders bevorzugt hat das wässrige Polymethylolgemisch folgende Zusammensetzung:
50 bis 80 Gew.-% Polymethylol (I),
0,1 bis 5 Gew.-% Methanol,
0,01 bis 5 Gew.-% tert. Amin,
0 bis 5 Gew.% organische Nebenverbindungen,
0,01 bis 5 Gew.% des Addukts aus tertiärem Amins und Ameisensäure (Amin-Formiat),
Rest Wasser.

Als organische Nebenverbindung kann beispielsweise die hydrierte Form des eingesetzten Alkanals enthalten sein, nämlich ein Alkohol der Formel (III) 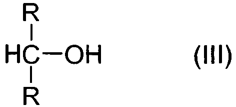 in der R jeweils unabhängig voneinander die oben genannte Bedeutung haben.

Das wässrige Polymethylolgemisch wird vorzugsweise aufgereinigt, in dem Leichtsieder von der Polymethylolverbindung abgetrennt werden.
Die Abtrennung der Leichtsieder von dem wässrigen Polymethylolgemisch erfolgt besonders bevorzugt durch Destillation (Stufe d)).

Die Destillation wird bevorzugt so durchgeführt, dass Leichtsieder, wie Wasser, Alkohol der Formel (III), Methanol und tertiäres Amin im Vakuum über Kopf abgetrennt werden.

Üblicherweise reagiert ein Teil der Amin-Formiate während der Destillation im Kolonnensumpf oder im Abtriebsteil der Kolonne mit Polymethylolverbindungen unter Bildung der freien Amine und der Formiate der Polymethylolverbindungen. Hierbei bildet sich bevorzugt der Monoester von Ameisensäure und der Polymethylolverbindung, welcher im Rahmen dieser Offenbarung als Polymethylol-Formiat bezeichnet wird.

Die durch die Umesterungsreaktion freigesetzten Amine werden in der Regel bei der Destillation zusammen mit den anderen Leichtsiedern am Kopf der Kolonne abgetrennt.
Die Destillation sollte deshalb so geregelt werden, dass die Konzentration der gebildeten Polymethylol-Formiate im Sumpfaustrag gering gehalten wird und das Zielprodukt, das Polymethylol, möglichst rein ist. Dies erfolgt bevorzugt dadurch, dass man bei der Destillation eine Sumpftemperatur oberhalb der Verdampfungstemperatur des Polymethylol-Formiats wählt, so dass die Polymethylol-Formiate vollständig oder weitesgehend vollständig durch Verdampfung in die Gasphase überführt werden.
Die durch diese Maßnahme bewirkte Verbesserung der Ausbeute und der Produktqualität ist wahrscheinlich darauf zurückzuführen, dass die Polymethyol-Formiate üblicherweise höher sieden als die anderen Leichtsieder und die Polymethyol-Formiate in der Regel deshalb im Verstärkungsteil der Kolonnen bei entsprechendem Rücklaufverhältnis niedergeschlagen werden. Die im Verstärkungsteil niedergeschlagenen Polymethylol-Formiate können mit Wasser hydrolysieren unter Rückbildung von Ameisensäure und der Polymethylolverbindung. Die Ameisensäure wird üblicherweise am Kolonnenkopf abgetrennt, während die Polymethylolverbindung in der Regel aus dem Kolonnensumpf ausgetragen werden kann.

In einer bevorzugten Ausführungsform wird deshalb die Destillation folgendermaßen durchgeführt:

Der Kondensator wird in der Regel bei einer Temperatur betrieben, in dem der überwiegende Teil der Leichtsieder bei dem entsprechenden Kopfdruck kondensiert wird. In der Regel liegt die Betriebstemperatur des Kondensators im Bereich von 0 bis 80°C, vorzugsweise 20 bis 50°C.
Als Kühlmedium kann hierbei bevorzugt möglichst kaltes Wasser (z. B. etwa 5°C) oder eine Kältemischung (z. B. Glykol-Wasser mit beispielsweise -20°C) verwendet werden. Der Kopfdruck beträgt besonders bevorzugt 0,001 bis 0,9 bar, besonders bevorzugt 0,01 bis 0,5 bar.
Das Vakuum wird üblicherweise großtechnisch mittels eines Dampfstrahlers erzeugt. Im Kolonnensumpf wird bevorzugt eine Temperatur eingestellt, die oberhalb der Verdampfungstemperatur des Polymethylol-Formiates liegt, so dass das Polymethylol-Formiat vollständig oder weitestgehend vollständig in die Gasphase übergeht. Besonders bevorzugt wird eine Temperatur eingestellt, die 5% bis 50% über der Siedetemperatur des Polymethylol-Formiats liegt und ganz besonders bevorzugt 10% bis 20% über der Siedetemperatur des Polymethylol-Formiats liegt.
Beispielsweise kann bei der Herstellung von NPG unter Verwendung von TMA als tertiäres Amin und einen Kolonnenkopfdruck von 175 mbar, bevorzugt eine Kolonnensumpftemperatur von 150 bis 170°C, besonders bevorzugt von 160 bis 165°C eingestellt werden.

Der Rücklauf am Kolonnenkopf wird in der Regel so eingestellt, dass die überwiegende Menge des Polymethylol-Formiats in der Kolonne zurückgehalten wird.
Vorzugsweise wird das am Kondensator anfallende Kondensat zu mehr als 20 Gew.-%, bevorzugt zu mehr als 30 Gew.-%, in die Destillationskolonne zurückgeführt. Dabei wird das Kondensat vorzugsweise in den Kolonnenkopf zurückgeführt.
Die für die Verdampfung erforderliche Energie wird üblicherweise durch einen Verdampfer im Kolonnensumpf eingetragen.
Dabei handelt es sich bei dem Verdampfer üblicherweise um einen Naturumlaufverdampfer oder Zwangsumlaufverdampfer. Es können aber auch Verdampfer mit kurzer Verweilzeit, Fallfilmverdampfer, Wendelrohrverdampfer, Wischfilmverdampfer oder ein Kurzwegverdampfer eingesetzt werden. Der Verdampfer kann in geeigneter Weise mit Wärme versorgt werden, beispielsweise mit 16 bar Dampf oder Wärmeträgeröl. Bevorzugt weist die Destillationskolonne Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Hold-up im Vergleich zu beispielsweise Ventilböden. Die Einbauten können in ein oder mehren Schüssen vorliegen.
Der Austrag aus der Hydrierung wird vorzugsweise in einem räumlichen Bereich zwischen ¼ und ¾ der theoretischen Böden der Destillationskolonne zugeführt, besonders bevorzugt in einem räumlichen Bereich zwischen 1/3 und 2/3 der theoretischen Böden der Destillationskolonne. Beispielsweise kann die Zuführung etwas oberhalb der Mitte der theoretischen Böden erfolgen (Verhältnis 3:4).
Die Anzahl der theoretischen Böden liegt im Allgemeinen im Bereich von 5 bis 30, vorzugsweise 10 bis 20.

Im Kondensator fällt als Kondensat ein Gemisch von Leichtsiedern an, das der Kolonne wie zuvor beschrieben, zum überwiegenden Teil als Rücklauf zugeführt wird. Beispielsweise kann das Leichtsiedergemisch tertiäres Amin, Methylolalkanal der Formel (II), nicht umgesetztes Aldehyd, Wasser sowie Alkohole der Formel (III), wie iso-Butanol aus Isobutyraldehyd oder n-Butanol aus n-Butyraldehyd, sowie Methanol aus Formaldehyd enthalten.

Erfindungssgemäß erfolgt die Rückgewinnung von Komponenten aus dem Leichtsiedergemisch, das bei der Destillation von Hydrierausträgen aus der Herstellung von Polymethylolen anfällt,
durch mehrstufige Destillation des Leichtsiedergemischs, enthaltend ein tertiäres Amin, Wasser, Methanol, Polymethylol der Formel (I), 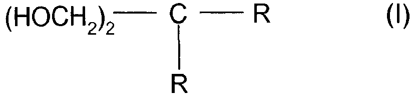 Methylolalkanal der Formel (II), 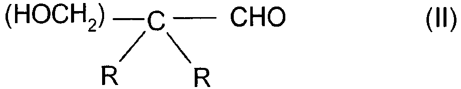 Alkohol der Formel (III)), 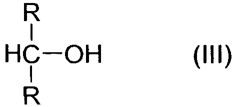 und ein Alkanal mit einer Methylengruppe in α-Stellung zur Carbonylgruppe,
und in der R jeweils unabhängig voneinander eine weitere Methylolgruppe oder eine Alkylgruppe mit 1 bis 22 C-Atomen oder eine Aryl- oder Aralkylgruppe mit 6 bis 22 C-Atomen bedeutet,
wobei in einer ersten Destillationsstufe eine Auftrennung des Leichtsiedergemischs in eine höhersiedende, überwiegend wasserreiche Fraktion und in eine das tertiäre Amin enthaltende leichtersiedende wässrige, organische Fraktion erfolgt,
und in der zweiten Destillationsstufe die wässrige, organische Fraktion aus der ersten Destillationsstufe in eine überwiegend aminhaltige Fraktion und eine weitere aminabgereicherte Fraktion aufgetrennt wird,
dadurch gekennzeichnet, dass das tertiäre Amin Trimethylamin oder Triethylamin ist und die Sumpftemperatur in der zweiten Destillationsstufe 110°C und mehr beträgt.

Das Leichtsiedergemisch, das in das erfindungsgemäße Verfahren eingesetzt wird, enthält vorzugsweise
1 bis 30 Gew.-% Methanol,
0,01 bis 1 Gew.-% nicht umgesetzter Aldehyd (Alkanal mit einer Methylengruppe in α-Stellung zur Carbonylgruppe),
0,1 bis 5 Gew.-% Alkohol (III),
0,1 bis 5 Gew.-% tert. Amin,
0,01 bis 5 Gew.-% Methylolalkanal (II),
0 bis 5 Gew.% organische Nebenverbindungen,
Rest Wasser.

Besonders bevorzugt enthält das Leichtsiedergemisch
1 bis 20 Gew.-% Methanol,
0,01 bis 1 Gew.-% nicht umgesetzter Aldehyd (Alkanal mit einer Methylengruppe in α-Stellung zur Carbonylgruppe),
0,5 bis 5 Gew.-% Alkohol (III),
0,5 bis 5 Gew.-% tert. Amin,
0,01 bis 1 Gew.-% Methylolalkanal (II),
0 bis 5 Gew.% organische Nebenverbindungen,
Rest Wasser.

Ein solches Leichtsiedergemisch wird bevorzugt durch mehrstufige Umsetzung von Alkanalen mit Formaldehyd erhalten.
Bevorzugt wird das Leichtsiedergemisch nach dem Hydrierverfahren erhalten.
Es ist jedoch auch möglich das erfindungsgemäße Verfahren zur Destillation eines Leichtsiedergemisches mit einem Leichtsiedergemisch durchzuführen, dass durch organische Canizzarro-Reaktion erhalten wurde

Wie obenstehend beschrieben, wird ein solches Leichtsiedergemisch vorzugsweise in einem mehrstufigen Hydrierverfahren erhalten, wobei man in Stufe a) Alkanale mit Formaldehyd in Gegenwart von tertiären Aminen als Katalysator zu Methylolalkanalen der Formel (II) kondensiert, und man anschließend in Stufe b) das aus Stufe a) erhaltenen Reaktionsgemisches in einen Sumpf, der überwiegend Verbindungen der Formel (II) enthält, und einen Kopfstrom, der Leichtsieder enthält, destillativ auftrennt, und man in Stufe c) den Sumpfaustrag aus Stufe b) hydriert und man anschließend in einer Stufe d) den Austrag aus Stufe c) destilliert, wobei das Leichtsiedergemisch aus Stufe d) abgetrennt wird.

In einer bevorzugten Ausführungsform wird dem Leichtsiedergemisch vor Zuführung in die erste Destillationsstufe eine Base zugesetzt.
Beispiele für eingesetzte Basen sind Alkalihydroxide, wie LiOH, NaOH oder KOH. Bevorzugt wird NaOH zugesetzt, besonders bevorzugt eine 25%-ige wässrige NaOH-Lösung.
Die Konzentration der zudosierten Base beträgt im allgemeinen im Bereich von 0,01 bis 2 Gew.-%, bevorzugt im Bereich von 0,5 bis 1 Gew.-%, besonders bevorzugt im Bereich von 0,2 bis 0,4 Gew.-%.
Durch die Zugabe der Base wird das im Kondensatstrom enthaltene Formiat-Addukt (Addukt aus tertiärem Amin und Ameisensäure) in der Regel in freies Amin und Natriumformiat gespalten. Durch diese Maßnahme kann die Ausbeute an zurück gewonnenem tertiärem Amin weiter erhöht werden.

Das Leichtsiedergemisch wird erfindungsgemäß einer mehrstufigen Destillation zugeführt.

Die Ausgestaltung der mehrstufigen Destillation ist in der Regel vom Siedepunkt des eingesetzten tertiären Amin abhängig.

Trimethylamin weist mit ca. 3°C bei Atmosphärendruck einen Siedepunkt auf, der niedriger ist als der Siedepunkt von Methanol (65°C).

Wird TMA als tertiäres Amin eingesetzt, so erfolgt die Destillation des Leichtsiedergemisches in der Regel in drei Stufen.

In der ersten Destillationsstufe ("Leichtsiederdestillation") erfolgt im Allgemeinen eine Auftrennung des Leichtsiedergemisches in eine wässrig-organische Fraktion, die am Kolonnenkopf entnommen wird, und eine überwiegend wasserreiche Fraktion, die aus dem Kolonnensumpf ausgetragen wird.

Der wässrige Austrag aus dem Kolonnensumpf, der in der Regel Alkaliformiat enthält, das durch Zugabe von Alkalihydroxiden gebildet wurde, wird in der Regel einer biologischen Abwasseraufbereitung zugeführt.

Die wässrige, organische Fraktion enthaltend Methylolalkanal der Formel (II), Alkohol der Formel (III), tertiäres Amin, Methanol, nicht umgesetztes Aldehyd und Reste von Wasser, wird im Allgemeinen in einer zweiten Destillationsstufe ("TMA-Destillation") destillativ in eine überwiegend aminhaltige Fraktion ("TMA-Fraktion"), die am Kolonnenkopf entnommen werden kann, und eine wässrige, organische (aminabgereicherte) Fraktion aufgetrennt, welche Methanol, Wasser und Alkohol der Formel (III) enthält. Erfindungsgemäß wird die "TMA-Destillation" so ausgeführt, dass die Sumpftemperatur während der Destillation 110°C und mehr beträgt.
Das am Kopf der Kolonne der zweiten Destillationsstufe anfallende Amin enthält vorzugsweise weniger als 2 Gew.-%, besonders bevorzugt weniger als 1 Gew.-%, Nebenprodukte, wie Methanol. Das so erhaltene TMA kann in den Prozess, beispielsweise in die erste Stufe des Hydrierverfahrens (Stufe a), Aldolreaktion) zurückgeführt werden und somit als basischer Katalysator in der Aldolreaktion eingesetzt werden.

Die wässrige, organische Fraktion aus dem Sumpf der zweiten Destillationsstufe ("TMA-Destillation") wird in der Regel in einer dritten Destillationsstufe ("MeOH-Destillation") in eine methanolische Phase aufgetrennt, die am Kolonnenkopf entnommen werden kann, und eine wässrig, organische Fraktion, die aus dem Kolonnensumpf ausgetragen wird.
Die methanolische Phase enthält überwiegend Methanol und vorzugsweise weniger als 2 Gew.-%, besonders bevorzugt weniger als 1 Gew.-% anderer Nebenkomponenten.
Die am Kolonnensumpf ausgeschleuste wässrig, organische Phase wird in der Regel in einem Wärmetauscher auf ca. 20 bis 50°C, vorzugsweise 25 bis 35°C, abgekühlt und in einem Phasenabscheider gesammelt. In dem Phasenscheider erfolgt üblicherweise eine Trennung in eine organische Phase, welche überwiegen Alkohol der Formel (III) enthält, sowie geringe Anteile von Methanol, Wasser und sonstigen organischen Komponenten enthalten kann. Die im Phasenscheider abgetrennte wässrige Lösung wird in der Regel der biologischen Abwasseraufbereitung zugeführt oder in die erste Destillationsstufe zurückgeführt. Die im Phasenscheider abgetrennte organische Phase wird im Allgemeinen ebenfalls ausgeschleust und in der Regel einer Verbrennung zugeführt.

Die "Leichtsieder-Destillation" wird bevorzugt bei folgenden Bedingungen ausgeführt:
Vorzugsweise wird das am Kondensator anfallende Kondensat zu mehr als 10 Gew.-%, besonders bevorzugt zu mehr als 50 Gew.-% in die Destillationskolonne zurückgeführt. Dabei wird das Kondensat vorzugsweise in den Kolonnenkopf zurückgeführt.

Der Kondensator wird bei einer Temperatur im Bereich von 5 bis 40°C, vorzugsweise 25 bis 35°C, insbesondere 30°C betrieben.

Der Kopfdruck liegt bevorzugt im Bereich von 0,8 bis 1,2 bar, besonders bevorzugt 0,9 bis 1,1 bar und insbesondere bevorzugt 1,02 bis 1,08 bar.

Als Kühlmedium kann hierbei bevorzugt möglichst kaltes Wasser (z. B. etwa 5°C) oder eine Kältemischung (z. B. Glykol-Wasser mit beispielsweise -20°C) verwendet werden.

Das Vakuum wird üblicherweise großtechnisch mittels eines Dampfstrahlers oder einer handelsüblichen Wasserstrahlpumpe erzeugt.

Die Temperatur des Kolonnensumpfes liegt im Bereich der Siedetemperatur von Wasser bei dem entsprechenden Druck. Sie kann knapp unterhalb aber auch knapp oberhalb der Siedetemperatur von Wasser liegen. Vorzugsweise beträgt die Temperatur des Kolonnensumpfes 80 bis 110% der Siedetemperatur von Wasser, besonders bevorzugt 90 bis 108% und ganz besonders bevorzugt 95 bis 105%.

Beispielsweise kann bei einem Kopfdruck von 1,05 bar vorteilhafterweise Temperatur des Kolonnensumpfes von 105°C eingestellt werden.

Die für die Verdampfung erforderliche Energie wird üblicherweise durch einen Verdampfer im Kolonnensumpf eingetragen.
Dabei handelt es sich bei dem Verdampfer bevorzugt um einen Naturumlaufverdampfer oder Zwangsumlaufverdampfer. Es können aber auch Verdampfer mit kurzer Verweilzeit, Fallfilmverdampfer, Wendelrohrverdampfer, Wischfilmverdampfer oder ein Kurzwegverdampfer eingesetzt werden.

Bevorzugt weist die Destillationskolonne Einbauten zur Erhöhung der Trennleistung auf.
Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Ebenso kann Ventilböden eingebaut werden. Die Einbauten können in ein oder mehren Schüssen unterteilt werden.
Der Kondensatstrom aus der Hydrierfeed-Destillation wird vorzugsweise in einem räumlichen Bereich zwischen ¼ und % der theoretischen Böden der Destillationskolonne zugeführt, besonders bevorzugt in einem räumlichen Bereich zwischen 1/3 und 2/3 der theoretischen Böden der Destillationskolonne, vorzugsweise bei 2/3 der theoretischen Böden. Ist beispielsweise die Kolonnenpackung in drei Schüsse unterteilt, so kann die Zuführung vorteilhafter weise oberhalb des 2. Schusses erfolgen.
Die Anzahl der theoretischen Böden liegt im Allgemeinen im Bereich von 5 bis 30, vorzugsweise 10 bis 20.

Vorzugsweise wird ein Austrag aus dem Sumpf des Verdampfers ausgeschleust, der überwiegend Wasser enthält, sowie ggf. Alkaliformiat, das durch Zugabe von Alkalihydroxiden gebildet wurde. Der Sumpfaustrag aus der "Leichtsieder-Destillation" wird in der Regel einer biologischen Abwasseraufbereitung zugeführt.

Im Kondensator fällt üblicherweise ein Kondensat an, das Wasser, Methanol, nicht umgesetzte Aldehyd, TMA und Alkohol der Formel (III) enthält, das als Kopfprodukt der "TMA-Destillation" zugeführt wird.

Die "TMA-Destillation" wird bevorzugt unter folgenden Bedingungen ausgeführt:
Vorzugsweise wird das am Kondensator anfallende Kondensat zu mehr als 30 Gew.-%, besonders bevorzugt zu mehr als 50 Gew.-% in die Destillationskolonne zurückgeführt. Dabei wird das Kondensat vorzugsweise in den Kolonnenkopf zurückgeführt.

Der Kondensator wird bei einer Temperatur im Bereich von 20 bis 40°C, vorzugsweise 25 bis 35°C, insbesondere 30°C betrieben.
Als Kühlmedium kann hierbei bevorzugt möglichst kaltes Wasser (z. B. etwa 5°C) oder eine Kältemischung (z. B. Glykol-Wasser mit beispielsweise -20°C) verwendet werden.

Der Kopfdruck liegt bevorzugt im Bereich von 3 bis 10 bar, besonders bevorzugt 4 bis 8 bar und insbesondere bevorzugt bei 6 bar absolut, bzw. 5 bar Überdruck.

Die Temperatur des Kolonnensumpfes beträgt erfindungsgemäß 110°C und mehr.

Vorzugsweise beträgt die Temperatur des Kolonnensumpfes 110 bis 160°C, besonders bevorzugt 110 bis 145°C und ganz besonders bevorzugt 130 bis 145°C. Beispielsweise kann bei einem Kopfdruck von 6 bar vorteilhafterweise eine Temperatur des Kolonnensumpfes von 140°C eingestellt werden.

Die für die Verdampfung erforderliche Energie wird üblicherweise durch einen Verdampfer in den Kolonnensumpf eingetragen.
Dabei handelt es sich bei dem Verdampfer bevorzugt um einen Naturumlaufverdampfer oder Zwangsumlaufverdampfer. Es können aber auch Verdampfer mit kurzer Verweilzeit, Fallfilmverdampfer, Wendelrohrverdampfer, Wischfilmverdampfer oder ein Kurzwegverdampfer eingesetzt werden.

Die "TMA-Destillation" wird bevorzugt in einer Bodenkolonne durchgeführt.

Bodenkolonnen werden für eine Vielzahl von Stoffaustauschprozessen zwischen Flüssigkeiten und Gasen verwendet. Dabei wird eine Flüssigkeit von oben nach unten zu einer Gasphase im Gegenstrom geführt. Das durch Öffnungen in den Kolonnenböden durchströmende Gas wird dabei in die Flüssigkeit eingespeist, so dass ein intensiver Stoffaustausch stattfindet. Die Flüssigkeit wird entweder durch spezielle Ableitvorrichtungen, insbesondere Bodenschächte (bei Querstromböden) oder über Öffnungen in den Kolonnenböden (bei Dualffowböden) auf den jeweils darunter liegenden Kolonnenboden weitergeleitet. Beispiel für unterschiedliche Bodentypen sind Siebböden, Dualflowböden, Glockenböden oder Ventilböden. Vorteilhaft erweisen sich die Böden bei der Reaktion des Aldehyds, da diese über hohe flüssige Hold-ups verfügen.

Die Anzahl der praktischen Böden liegt im Allgemeinen im Bereich von 10 bis 100, vorzugsweise 20 bis 90, besonders bevorzugt 30 bis 40.

In einer ganz besonders bevorzugten Ausführungsform wird die Anzahl der Böden wird so gewählt, so dass die Verweilzeit in der Kolonne 5 Minuten und mehr, bevorzugt 7 Minuten und mehr und besonders bevorzugt 10 Minuten und mehr beträgt.
Bevorzugt liegt die Verweilzeit im Bereich von 5 bis 45 Minuten, bevorzugt im Bereich von 7 bis 30 Minuten und besonders bevorzugt im Bereich von 8 bis 20 Minuten.
Die Verweilzeit in der Kolonne wird offenbarungsgemäß berechnet, in dem das Volumen des Flüssigkeits-Hold-ups der trennwirksamen Einbauten zwischen Sumpf und Zuführung des Feeds (V_{Hold-up}) durch die Summe aus Rücklauf und Zulaufvolumenstrom der Kolonne dividiert wird (Verweilzeit = V_{Hold-up}/(Zulaufstrom + Rücklaufstrom)). Wenn beispielsweise die trennwirksamen Einbauten Böden sind, wie es in der zuvor beschriebenen bevorzugten Ausführungsform der Fall ist, ist das Volumen des Flüssigkeits-Hold-ups der trennwirksamen Einbauten zwischen Sumpf und Zuführung des Feeds üblicherweise das Flüssigkeitsvolumen auf den entsprechenden Böden.

Im Rahmen der vorliegenden Erfindung kann das Volumen V_{Hold-up} auch das Volumen des Kolonnensumpf (V_{Sumpf}) und/oder das Volumen eines vorgeschalteten Temper-Reaktors V_{Temper-Reaktor} mit umfassen, wenn die Temperatur der Flüssigkeit in diesen Volumina 110°C und mehr beträgt. Im Rahmen dieser Erfindung wurde gefunden, dass eine weitere Verbesserung der Abtrennung des tertiären Amins möglich ist, wenn die Verweilzeit bei hohen Temperaturen ausreichend lang ist. Dies ist wahrscheinlich darauf zurückzuführen, dass die in der Aldolreaktion nicht umgesetzten Aldehyde die Flüchtigkeit der tertiären Amine reduzieren und somit die Abtrennung erschweren. Eine mögliche Erklärung für die verbesserte Abtrennung ist, dass bei der erfindungsgemäßen Temperatur von 110°C und mehr die Aldehyde zu höher siedenden Nebenprodukten reagieren, welche die Abtrennung des tertiären Amins nicht mehr behindern. Somit kann anstatt der Erhöhung der Verweilzeit im heißen Teil der Kolonne, zum Beispiel durch die Erhöhung des Flüssigkeitsvolumen der trennwirksamen Einbauten (höheres Volumen pro Boden oder Erhöhung der Anzahl von Böden), die Verweilzeit auch dadurch verlängert werden, dass der Destillationskolonne ein Temper-Reaktor vorgeschaltet wird oder der Kolonnensumpf vergrößert wird.
In einer weiteren besonders bevorzugten Ausführungsform beträgt deshalb die Verweilzeit bei Temperaturen von 110°C und mehr in der zweiten Destillationsstufe bevorzugt 5 Minuten und mehr und besonders bevorzugt 10 Minuten und mehr.
Bevorzugt liegt die Verweilzeit bei Temperaturen von 110°C und mehr in der zweiten Destillationsstufe im Bereich von 5 bis 45 Minuten, bevorzugt im Bereich von 7 bis 30 Minuten und besonders bevorzugt im Bereich von 10 bis 20 Minuten.
In dieser bevorzugten Ausführungsform ist die Verweilzeit definiert als das Flüssigkeitsvolumen der zweiten Kolonne, welches eine Temperatur von 110°C und mehr aufweist (V_{Flüssig}), dividiert durch die Summe aus Rücklauf und Zulaufvolumenstrom der Kolonne (Verweilzeit = V_{Flüssig}/(Zulaufstrom + Rücklaufstrom)), wobei das Flüssigkeitsvolumen der zweiten Kolonne (V_{Flüssig}) die Summe aus V_{Hold-up}, V_{Temper-Reaktor} und V_{Sumpf} ist (V_{Flüssig}= V_{Hold-up} + V_{Temper-Reaktor} + V_{Sumpf}). In dieser besonders bevorzugten Ausführungsform umfasst definitionsgemäß nur die Flüssigkeitsvolumina, die eine Temperatur von 110°C und mehr aufweisen. Flüssigkeitsvolumina, die eine Temperatur von weniger als 110°C aufweisen, werden für die Berechnung der Verweilzeit in dieser besonders bevorzugten Ausführungsform nicht berücksichtigt.

Der Austrag aus der "Leichtsieder-Destillation" wird vorzugsweise in einem räumlichen Bereich zwischen ¼ und ¾ der praktischen Böden der Destillationskolonne zugeführt, besonders bevorzugt in einem räumlichen Bereich zwischen 1/3 und 2/3, vorzugsweise zwischen ½ und 2/3 der praktischen Böden der Destillationskolonne, vorzugsweise bei 2/3 der praktischen Böden. Beispielsweise erfolgt in einer Kolonne mit 60 praktischen Böden die Zuführung bevorzugt oberhalb des 35. Boden von unten.

In einer alternativen Ausführungsform weist die Destillationskolonne Einbauten zur Erhöhung der Trennleistung auf.

Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Die Einbauten können in ein oder mehren Schüssen unterteilt werden.

Im Kondensator fällt üblicherweise ein Kondensat an, das überwiegend TMA enthält vorzugsweise mit weniger als 2 Gew.-%, besonders bevorzugt mit weniger als 1 Gew.-%, Nebenprodukten, wie Methanol. Das so erhaltene TMA kann in die PolymethylolHerstellung, beispielsweise in die erste Stufe des Hydrierverfahrens (Stufe a), Aldolreaktion) zurückgeführt werden.

Vorzugsweise wird ein wässrig, organischer Austrag aus dem Sumpf des Verdampfers ausgeschleust, der Methylolalkanal der Formel (II), Alkohol der Formel (III), Methanol, Spruen von Aldehyd und Wasser enthält.
Der Sumpfaustrag wird in der Regel der "MeOH-Destillation" zugeleitet.

Die "MeOH-Destillation" wird bevorzugt unter folgenden Bedingungen ausgeführt:
Vorzugsweise wird das am Kondensator anfallende Kondensat zu mehr als 30 Gew.-%, besonders bevorzugt zu mehr als 50 Gew.-% in die Destillationskolonne zurückgeführt. Dabei wird das Kondensat vorzugsweise in den Kolonnenkopf zurückgeführt.

Der Kondensator wird bei einer Temperatur im Bereich von 20 bis 40°C, vorzugsweise 25 bis 35°C, insbesondere 30°C betrieben.

Als Kühlmedium kann hierbei bevorzugt möglichst kaltes Wasser (z. B. etwa 5°C) oder eine Kältemischung (z. B. Glykol-Wasser mit beispielsweise -20°C) verwendet werden.

Der Kopfdruck liegt bevorzugt im Bereich von 0,8 bis 1,2 bar, besonders bevorzugt 0,9 bis 1,1 bar und insbesondere bevorzugt 1,02 bis 1,08 bar.

Das Vakuum wird üblicherweise großtechnisch mittels eines Dampfstrahlers oder einer handelsüblichen Wasserstrahlpumpe erzeugt.

Die Temperatur des Kolonnensumpfes wird in der Regel so eingestellt, dass Methanol in den gasförmigen Zustand überführt wird.
Vorzugsweise beträgt die Temperatur des Kolonnensumpfes 100 bis 300% der Siedetemperatur von Methanol, besonders bevorzugt 100 bis 175% und ganz besonders bevorzugt 110 bis 150%.

Beispielsweise kann bei einem Kopfdruck von 1,05 bar bevorzugt eine Temperatur des Kolonnensumpfes von 92°C eingestellt werden, wobei die Temperatur einem Wert von ca. 150% der Siedetemperatur von Methanol bei diesem Druck entspricht.

Die für die Verdampfung erforderliche Energie wird üblicherweise durch einen Verdampfer im Kolonnensumpf eingetragen.
Dabei handelt es sich bei dem Verdampfer bevorzugt um einen Naturumlaufverdampfer oder Zwangsumlaufverdampfer. Es können aber auch Verdampfer mit kurzer Verweilzeit, Fallfilmverdampfer, Wendelrohrverdampfer, Wischfilmverdampfer oder ein Kurzwegverdampfer eingesetzt werden.
Bevorzugt weist die Destillationskolonne Einbauten zur Erhöhung der Trennleistung auf.
Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Holdup im Vergleich zu beispielsweise Ventilböden.
Die Einbauten können in ein oder mehren Schüssen unterteilt werden.

Der Sumpfstrom aus der "TMA-Destillation" wird vorzugsweise in einem räumlichen Bereich zwischen ¼ und ¾ der praktischen Böden der Destillationskolonne zugeführt, besonders bevorzugt in einem räumlichen Bereich zwischen 1/3 und 2/3 der praktischen Böden der Destillationskolonne, vorzugsweise bei 2/3 der praktischen Böden. Ist beispielsweise die Kolonnenpackung in drei Schüsse unterteilt, so kann die Zuführung vorteilhafter weise oberhalb des 2. Schusses erfolgen.
Die Anzahl der praktischen Böden liegt im Allgemeinen im Bereich von 5 bis 50, vorzugsweise 20 bis 30.

Im Kondensator fällt üblicherweise ein Kondensat (methanolische Phase) an. Die methanolische Phase enthält überwiegend Methanol und vorzugsweise weniger als 2 Gew.-%, besonders bevorzugt weniger als 0,15 Gew.-% anderer Nebenkomponenten. Die so erhaltene methanolische Phase kann als Ausgangsstoff oder Lösungsmittel in organischen Synthesen eingesetzt werden.

Vorzugsweise wird ein wässrig, organische Phase aus dem Sumpf des Verdampfers ausgeschleust, welche überwiegend Alkohol der Formel (III) enthält, sowie geringe Anteile von Methanol, Wasser und sonstigen organischen Komponenten enthalten kann.
Die am Kolonnensumpf ausgeschleuste wässrig, organische Phase wird in der Regel in einem Wärmetauscher auf ca. 20 bis 50°C, vorzugsweise 25 bis 35°C, abgekühlt und in einem Phasenabscheider gesammelt. In dem Phasenscheider erfolgt üblicherweise eine Trennung in eine organische Phase und eine wässrige Phase.
Die im Phasenscheider abgetrennte wässrige Lösung wird in der Regel der biologischen Abwasseraufbereitung zugeführt oder in "Leichtsieder-Destillation" zurückgeführt.
Die im Phasenscheider abgetrennte organische Phase wird im Allgemeinen ebenfalls ausgeschleust und in der Regel einer Verbrennung zugeführt.

In einer besonderen Ausführungsform werden die "TMA-Destillation" und die "MeOH-Destillation" in einer einzigen Stufe gemeinsam in einer Trennwandkolonne durchgeführt.

In einer Trennwandkolonne ist üblicherweise eine Trennwand in Kolonnenlängsrichtung unter Ausbildung eines oberen gemeinsamen Kolonnenbereiches, eines unteren gemeinsamen Kolonnenbereiches, eines Zulaufteils mit Verstärkungsteil und Abtriebsteil sowie eines Entnahmeteils mit Verstärkungsteil und Abtriebsteil angeordnet. Der Aufbau einer typischen Trennwandkolonne ist beispielsweise in EP 0122 367 beschrieben.

Die Trennwandkolonne ist vorzugsweise als Bodenkolonne ausgestaltet.
Die Anzahl der praktischen Böden beträgt in der Regel zwischen 20 und 100, vorzugsweise zwischen 30 und 80 und vorzugsweise zwischen 50 und 70.
Das Trennblech verläuft üblicherweise oberhalb des ersten Viertels der praktischen Böden bis oberhalb des dritten Viertel der praktischen Böden. Bei einer praktischen Bodenanzahl von 60 verläuft beispielsweise das Trennblech vorzugsweise vom 15. bis zum 45. Boden.

Das Kopfprodukt aus der "Leichtsieder-Destillation" wird auf der Zulaufseite der Trennwandkolonne eingetragen, vorzugsweise in einem Bereich zwischen 1/3 und 2/3 der Gesamtanzahl der praktischen Böden, vorzugsweise auf Höhe der Hälfte der praktischen Böden.

Die Destillation wird vorzugsweise bei erhöhtem Druck, vorzugsweise bei einem Druck von 1 bis 10 bar, vorzugsweise 2 bis 8 bar, besonders bevorzugt 3 bis 7 bar, insbesondere 5 bar, durchgeführt.

Die Temperatur des Kolonnensumpfes beträgt erfindungsgemäß 110°C und mehr. Vorzugsweise beträgt die Temperatur des Kolonnensumpfes 110 bis 160°C, besonders bevorzugt 110 bis 145°C und ganz besonders bevorzugt 120 bis 140°C. Beispielsweise kann bei einem Kopfdruck von 5 bar vorteilhafterweise Temperatur des Kolonnensumpfes von 130°C eingestellt werden.

Die für die Verdampfung erforderliche Energie wird üblicherweise durch einen Verdampfer im Kolonnensumpf eingetragen.
Dabei handelt es sich bei dem Verdampfer bevorzugt um einen Naturumlaufverdampfer oder Zwangsumlaufverdampfer. Es können aber auch Verdampfer mit kurzer Verweilzeit, Fallfilmverdampfer, Wendelrohrverdampfer, Wischfilmverdampfer oder ein Kurzwegverdampfer eingesetzt werden.

Der Kondensator wird bei einer Temperatur im Bereich von 20 bis 40°C, vorzugsweise 25 bis 35°C, insbesondere 30°C betrieben.

Als Kühlmedium kann hierbei bevorzugt möglichst kaltes Wasser (z. B. etwa 5°C) oder eine Kältemischung (z. B. Glykol-Wasser mit beispielsweise -20°C) verwendet werden.

Vorzugsweise wird das am Kondensator anfallende Kondensat zu mehr als 30 Gew.-%, besonders bevorzugt zu mehr als 50 Gew.-%, insbesondere bevorzugt mehr als 75 Gew.-% in die Destillationskolonne zurückgeführt. Dabei wird das zurückgeführte Kondensat vorzugsweise zu 10 bis 50% auf die Zulaufseite und zu 50 bis 90% auf die Entnahmeseite aufgeteilt.

In einer ganz besonders bevorzugten Ausführungsform beträgt die Verweilzeit in der Kolonne 5 Minuten und mehr, bevorzugt 7 Minuten und mehr und besonders bevorzugt 10 Minuten und mehr beträgt.
Bevorzugt liegt die Verweilzeit im Bereich von 5 bis 45 Minuten, bevorzugt im Bereich von 7 bis 30 Minuten und besonders bevorzugt im Bereich von 8 bis 20 Minuten.
Die Verweilzeit in der Trennwandkolonne wird offenbarungsgemäß berechnet, in dem das Volumen des Flüssigkeits-Hold-ups der trennwirksamen Einbauten unterhalb des unteren Ende des Trennbleches und oberhalb des Sumpfes(V_{Hold-up}) durch die Summe aus Rücklauf und Zulaufvolumenstrom der Kolonne dividiert wird (Verweilzeit = V_{Holdup}/(Zulaufstrom + Rücklaufstrom)).
Wenn beispielsweise die trennwirksamen Einbauten Böden sind, wie es in der zuvor beschriebenen bevorzugten Ausführungsform der Fall ist, ist das Volumen des Flüssigkeits-Hold-ups der trennwirksamen Einbauten zwischen Sumpf und Zuführung des Feeds üblicherweise das Flüssigkeitsvolumen auf den entsprechenden Böden.

Im Rahmen der vorliegenden Erfindung kann das Volumen V_{Hold-up} auch das Volumen des Kolonnensumpf (V_{Sumpf}) und/oder das Volumen eines vorgeschalteten Temper-Reaktors V_{Temper-Reaktor} mit umfassen, wenn die Temperatur der Flüssigkeit in diesen Volumina 110°C und mehr beträgt. Im Rahmen dieser Erfindung wurde gefunden, dass eine weitere Verbesserung der Abtrennung des tertiären Amins möglich ist, wenn die Verweilzeit bei hohen Temperaturen ausreichend lang ist. Dies ist wahrscheinlich dar- auf zurückzuführen, dass die in der Aldolreaktion nicht umgesetzten Aldehyde die Flüchtigkeit der tertiären Amine reduzieren und somit die Abtrennung erschweren. Eine mögliche Erklärung für die verbesserte Abtrennung ist, dass bei der erfindungsgemäßen Temperatur von 110°C und mehr die Aldehyde zu höher siedenden Nebenprodukten reagieren, welche die Abtrennung des tertiären Amins nicht mehr behindern. Somit kann anstatt der Erhöhung der Verweilzeit im heißen Teil der Kolonne, zum Beispiel durch die Erhöhung des Flüssigkeitsvolumen der trennwirksamen Einbauten (höheres Volumen pro Boden oder Erhöhung der Anzahl von Böden), die Verweilzeit auch dadurch verlängert werden, dass der Destillationskolonne ein Temper-Reaktor vorgeschaltet wird oder der Kolonnensumpf vergrößert wird.
In einer weiteren besonders bevorzugten Ausführungsform beträgt deshalb die Verweilzeit bei Temperaturen von 110°C und mehr in der zweiten Destillationsstufe bevorzugt 5 Minuten und mehr und besonders bevorzugt 10 Minuten und mehr.
Bevorzugt liegt die Verweilzeit bei Temperaturen von 110°C und mehr in der zweiten Destillationsstufe im Bereich von 5 bis 45 Minuten, bevorzugt im Bereich von 7 bis 30 Minuten und besonders bevorzugt im Bereich von 10 bis 20 Minuten.
In dieser bevorzugten Ausführungsform ist die Verweilzeit definiert als das Flüssigkeitsvolumen der zweiten Kolonne, welches eine Temperatur von 110°C und mehr aufweist (V_{Flüssig}), dividiert durch die Summe aus Rücklauf und Zulaufvolumenstrom der Kolonne (Verweilzeit = V_{Flüssig}/(Zulaufstrom + Rücklaufstrom)), wobei das Flüssigkeitsvolumen der zweiten Kolonne (V_{Flüssig}) die Summe aus V_{Hold-up}, V_{Temper-Reaktor} und V_{Sumpf} ist (V_{Flüssig}= V_{Hold-up} + V_{Temper-Reaktor} + V_{Sumpf}). In dieser besonders bevorzugten Ausführungsform umfasst umfasst definitionsgemäß nur die Flüssigkeitsvolumina, die eine Temperatur von 110°C und mehr aufweisen. Flüssigkeitsvolumina, die eine Temperatur von weniger als 110°C aufweisen, werden für die Berechnung der Verweilzeit in dieser besonders bevorzugten Ausführungsform nicht berücksichtigt.

Das am Kolonnenkopf entnommene Kondensat enthält überwiegend TMA, vorzugsweise zu mehr als 95 Gew.-%, besonders bevorzugt zu mehr als 98 Gew.-% TMA. Als Nebenkomponenten kann Methanol vorhanden sein.
Das so erhaltene TMA kann in die erste Stufe des Hydrierprozesses (Stufe a), Aldolreaktion) zurückgeführt werden.

Auf der Entnahmeseite wird eine methanolische Fraktion entnommen.
Die methanolische Fraktion wird vorzugsweise in einem räumlichen Bereich zwischen 1/6 und 1/2 , vorzugsweise 1/5 und 1/3, insbesondere ¼ und 1/3 der praktischen Böden der Destillationskolonne entnommen. Die methanolische Fraktion enthält vorzugsweise mehr als 99 Gew.-% Methanol, besonders bevorzugt mehr als 99,5 Gew.-% Methanol und ganz besonders bevorzugt mehr als 99,8 Gew.-% Methanol.

Aus dem Kolonnensumpf wird in der Regel eine wässrig, organische Phase ausgeschleust, welche überwiegend Alkohol der Formel (III) enthält, sowie geringe Anteile von Methanol, Wasser und sonstigen organischen Komponenten enthalten kann.

Die am Kolonnensumpf ausgeschleuste wässrig, organische Phase wird in der Regel in einem Wärmetauscher auf ca. 20 bis 50°C, vorzugsweise 25 bis 35°C, abgekühlt und in einem Phasenabscheider gesammelt. In dem Phasenscheider erfolgt üblicherweise eine Trennung in eine organische Phase und eine wässrige Phase. Die im Phasenscheider abgetrennte wässrige Lösung wird in der Regel der biologischen Abwasseraufbereitung zugeführt oder in die erste Destillationsstufe ("Leichtsiederdestillation") zurückgeführt.
Die im Phasenscheider abgetrennte organische Phase wird im Allgemeinen ebenfalls ausgeschleust und in der Regel einer Verbrennung zugeführt.

Wird TEA als tertiäres Amin eingesetzt, so erfolgt die Destillation des Leichtsiedergemisches in der Regel ebenfalls in drei Stufen. Bei Atmosphärendruck weist TEA einen Siedepunkt von ca. 89°C auf, der unterhalb des Siedepunktes von Wasser (100°C) aber oberhalb des Siedepunktes von Methanol (ca. 65°C) liegt.

Die destillative Aufarbeitung des Leichtsiedergemisches erfolgt in der Regel in drei Stufen.

In der ersten Destillationstufe ("Leichtsiederdestillation") erfolgt im Allgemeinen eine Auftrennung des Leichtsiedergemisches in eine wässrig-organische Fraktion, die am Kolonnenkopf entnommen wird, und eine überwiegend wasserreiche Fraktion, die aus dem Kolonnensumpf ausgetragen wird.

Der wässrige Austrag aus dem Kolonnensumpf, der in der Regel Alkaliformiat enthält, das durch Zugabe von Alkalihydroxiden gebildet wurde, wird in der Regel einer biologischen Abwasseraufbereitung zugeführt.

Die wässrige, organische Fraktion enthaltend Methylolalkanal der Formel (II), Alkohol der Formel (III), TEA, Methanol, nicht umgesetztes Aldehyd und Wasser, wird im Allgemeinen in einer zweiten Destillationsstufe ("TEA-Destillation") destillativ in eine überwiegend aminhaltige Fraktion ("TEA-Fraktion"), die den größten Teil des MeOH enthält und die am Kolonnenkopf entnommen werden kann, und eine wässrige, organische (aminabgereicherte) Fraktion aufgetrennt, welche überwiegend Wasser und Alkohol der Formel (III), sowie Reste von MeOH enthält.
Erfindungsgemäß wird die "TEA-Destillation" so ausgeführt, dass die Sumpftemperatur während der Destillation 100°C und mehr beträgt.
Das am Kopf der Kolonne anfallende Amin enthält größere Mengen an MeOH, vorzugsweise beträgt der Anteil von MeOH im Kondensat im Bereich von 30 bis 90 Gew.-%, besonders bevorzugt im Bereich von 40 bis 85 Gew.-% und insbesondere bevorzugt 50 bis 75 Gew.-%.

Das Kopfprodukt aus der der zweiten Destillationsstufe ("TEA-Destillation") wird in der Regel in einer dritten Destillationsstufe ("MeOH-Destillation") in eine methanolische Phase aufgetrennt, die am Kolonnenkopf entnommen werden kann, und eine Fraktion, die TEA enthält, die aus dem Kolonnensumpf ausgetragen wird.
Die methanolische Phase enthält überwiegend Methanol und vorzugsweise weniger als 3 Gew.-%, besonders bevorzugt weniger als 2 Gew.-%, insbesondere bevorzugt weniger als 1 Gew.-% anderer Nebenkomponenten.

Die erste Stufe ("Leichtsiederdestillation") wird vorzugsweise unter denselben Bedingungen durchgeführt, wie sie in der "Leichtsiederdestillation" bei der Verwendung von TMA beschrieben wurde.

Die zweite Stufe ("TEA-Destillation") wird bevorzugt unter den Bedingungen durchgeführt, wie sie zuvor in der "TMA-Destillation" beschrieben wurden.
Unter diesen Bedingungen fällt im Kondensator üblicherweise ein Kondensat an, das überwiegend MeOH und TEA, sowie Reste von Wasser enthält.
Üblicherweise beträgt der Anteil an MeOH im Kondensat im Bereich von 30 bis 90 Gew.-%, besonders bevorzugt im Bereich von 40 bis 85 Gew.-% und insbesondere bevorzugt 50 bis 80 Gew.-%.
Der Anteil an TEA beträgt bevorzugt 10 bis 70 Gew.-%, besonders bevorzugt 15 bis 60 Gew.-%, insbesondere bevorzugt 20 bis 50 Gew.-%
Der Anteil von Wasser beträgt üblicherweise weniger als 10 Gew.-%, bevorzugt weniger als 6 Gew.-% und besonders bevorzugt weniger als 4 Gew.-%.
Aus dem Sumpf des Verdampfers wird ein Strom ausgeschleust, der neben Wasser in der Regel Alkohol der Formel (III) enthält. Dieser Strom wird vorzugsweise gekühlt und in einem Phasenscheider in eine organische Phase, die überwiegend den Alkohol der Formel (III) enthält, und eine wässrige Phase getrennt.
Der Kondensatstrom aus der zweiten Stufe ("TEA-Destillation") wird vorzugsweise einer dritten Stufe ("MeOH-Destillation") zugeführt.

Die "MeOH-Destillation" wird bevorzugt unter folgenden Bedingungen ausgeführt:
Vorzugsweise wird das am Kondensator anfallende Kondensat zu mehr als 30 Gew.-%, besonders bevorzugt zu mehr als 50 Gew.-% in die Destillationskolonne zurückgeführt. Dabei wird das Kondensat vorzugsweise in den Kolonnenkopf zurückgeführt.

Der Kondensator wird bei einer Temperatur im Bereich von 20 bis 40°C, vorzugsweise 25 bis 35°C, insbesondere 30°C betrieben.

Als Kühlmedium kann hierbei bevorzugt möglichst kaltes Wasser (z. B. etwa 5°C) oder eine Kältemischung (z. B. Glykol-Wasser mit beispielsweise -20°C) verwendet werden.

Der Kopfdruck liegt bevorzugt im Bereich von 0,8 bis 1,2 bar, besonders bevorzugt 0,9 bis 1,1 bar und insbesondere bevorzugt 1,02 bis 1,08 bar.

Das Vakuum wird üblicherweise großtechnisch mittels eines Dampfstrahlers oder einer handelsüblichen Wasserstrahlpumpe erzeugt.

Die Temperatur des Kolonnensumpfes wird in der Regel so eingestellt, dass Methanol in den gasförmigen Zustand überführt wird.
Vorzugsweise beträgt die Temperatur des Kolonnensumpfes 100 bis 300% der Siedetemperatur von Methanol, besonders bevorzugt 100 bis 175% und ganz besonders bevorzugt 105 bis 150%.
Beispielsweise kann bei einem Kopfdruck von 1,05 bar bevorzugt eine Temperatur des Kolonnensumpfes von 75°C eingestellt werden, wobei die Temperatur einem Wert von ca. 115% der Siedetemperatur von Methanol bei diesem Druck entspricht.

Die für die Verdampfung erforderliche Energie wird üblicherweise durch einen Verdampfer im Kolonnensumpf eingetragen.
Dabei handelt es sich bei dem Verdampfer bevorzugt um einen Naturumlaufverdampfer oder Zwangsumlaufverdampfer. Es können aber auch Verdampfer mit kurzer Verweilzeit, Fallfilmverdampfer, Wendelrohrverdampfer, Wischfilmverdampfer oder ein Kurzwegverdampfer eingesetzt werden.
Bevorzugt weist die Destillationskolonne Einbauten zur Erhöhung der Trennleistung auf.
Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten ist der geringe Druckverlust und der geringe spezifische Flüssig-Holdup im Vergleich zu beispielsweise Ventilböden.
Die Einbauten können in ein oder mehren Schüssen unterteilt werden.

Der Sumpfstrom aus der "TMA-Destillation" wird vorzugsweise in einem räumlichen Bereich zwischen ¼ und ¾ der praktischen Böden der Destillationskolonne zugeführt, besonders bevorzugt in einem räumlichen Bereich zwischen 1/3 und 2/3 der praktischen Böden der Destillationskolonne, vorzugsweise bei 2/3 der praktischen Böden. Ist beispielsweise die Kolonnenpackung in drei Schüsse unterteilt, so kann die Zuführung vorteilhafter weise oberhalb des 2. Schusses erfolgen.
Die Anzahl der praktischen Böden liegt im Allgemeinen im Bereich von 5 bis 50, vorzugsweise 20 bis 30.

Im Kondensator fällt üblicherweise ein Kondensat an, das überwiegend Methanol, enthält. Die methanolische Phase enthält überwiegend Methanol und vorzugsweise weniger als 2 Gew.-%, besonders bevorzugt weniger als 0,15 Gew.-% anderer Nebenkomponenten. Die so erhaltene methanolische Phase kann als Ausgangsstoff oder Lösungsmittel in organischen Synthesen eingesetzt werden.

Vorzugsweise wird ein Rückstand aus dem Sumpf des Verdampfers ausgeschleust, welche überwiegend TEA enthält, sowie geringe Anteile von Methanol, Wasser und sonstigen organischen Komponenten.
Der Gehalt an TEA im Sumpfaustrag beträgt vorzugsweise mehr als 60 Gew.-%, vorzugsweise mehr als 70 Gew.-% und besonders bevorzugt mehr als 80 Gew.-%.

Die am Kolonnensumpf ausgeschleuste TEA wird in der Regel in einem Wärmetauscher auf ca. 10 bis 50°C, vorzugsweise 15 bis 25°C, abgekühlt und in einem Phasenabscheider gesammelt. In dem Phasenscheider erfolgt üblicherweise eine Trennung in eine organische Phase, die überwiegend TEA enthält und eine wässrig, methanolische Phase. Die wässrig, methanolische Phase kann verworfen oder der biologischen Abwasseraufbereitung zugeführt werden.
Die im Phasenscheider abgetrennte TEA-Phase wird im Allgemeinen in die Aldolreaktion zurückgeführt.

Durch erfindungsgemäße Trennung der anfallenden Nebenprodukte und Produkte, insbesondere durch die Rückführbarkeit des tertiären Amins in die Aldolreaktion, kann die Wirtschaftlichkeit des Verfahrens weiter verbessert werden, da die meisten Komponenten stofflich verwertet werden können, beispielsweise durch Rückführung in den Prozess. Der Anteil an Verbindungen, die einer Entsorgung zugeführt werden müssen, werden verringert, so dass die Entsorgungskosten bei dem erfindungsgemäßen Verfahren reduziert werden können.
Das bei der erfindungsgemäßen destillativen Aufarbeitung des Leichtsiedergemisches zurück gewonnene tertiäre Amin kann als Katalysator in die Polymethylolherstellung, zum Beispiel in die Aldolreaktion (Umsetzung von Formaldehyd mit Aldehyden), zurückgeführt werden, da es lediglich einen geringen Anteil an Alkoholen, wie Methanol oder n-Butanol aufweist. Gerade diese Alkohole sind nämlich für die Rückführbarkeit nachteilig, da diese Alkohole Nebenreaktionen in der Aldolreaktion eingehen, welche die Ausbeute verringern. Zudem stören solche Alkohole bei der nachfolgenden Abtrennung von nicht umgesetzten Aldehyden aus dem der Austrag der Aldolreaktion, da sie ähnliche Siedepunkte aufweisen und mit den nicht umgesetzten Aldehyden Azeotrope und oder die Trennung behindernde Halbacetale bilden können. Durch das erfindungsgemäße Verfahren werden diese Nachteile weitestgehend vermieden.

Somit kommt es nicht zu einer Aufpegelung von Methanol in der Aldolreaktion.

Die anderen Komponenten des Leichtsiedergemischs, wie MeOH, können ebenfalls in einer hohen Reinheit gewonnen werden, so dass die Verwertung dieses Stoffes möglich ist, beispielsweise als Ausgangsmaterial oder Lösungsmittel für organische Synthesen, wie der Formaldehydherstellung.
Weiterhin konnte der apparatetechnischen Aufwand für die Destillation so gering wie möglich zu halten, um ein wirtschaftliches Aufarbeitungsverfahren zur Verfügung stellen zu können.

Die Erfindung wird anhand der folgenden Beispiele erläutert:

### Herstellung von Roh-Polymethylol mit dem Hydrierverfahren

### Stufe a) Aldolreaktion:

Es wurde ca. 750 g/h Isobutyraldehyd (ca. >99,5 GC-Flächen-% IBA) mit ca. 700 g/h Formaldehyd (ca. 49 Gew.-% Formaldehyd, 1,5 Gew.-% Methanol, Rest Wasser) und 40 g/h Trimethylaminlösung (50 Gew.-% TMA in Wasser) in einer zweistufigen Rührkesselkaskade zur Reaktion gebracht.

### Stufe b) Destillative Auftrennung des Reaktionsgemisches aus Stufe a):

Anschließend wurde der Reaktionsautrag aus der Aldolreaktion in einer Kolonne destillativ von Leichtsiedern befreit. Die Kolonne war mit 1,5 m Gewebepackung (500m²/m³ spezifische Oberfläche) im Verstärkungsteil und 4 m Blechpackung (250 m²/m³) ausgerüstet. Der Aldolisierungsaustrag wurde oberhalb der Blechpackung zugeführt. Am Kopf der Kolonne wurde ein Kondensator mit Kühlwasser (ca. 10°C) und einem nachgeschalteten Phasenscheider verwendet. Am Kopf wurde das Destillat gasförmig dem Kondensator zugeführt. Es fielen ca. 255 g/h flüssiges Kondensat an. In dem nach geschalteten Phasenscheider wurde eine wässrige Phase von 95 g/h abgetrennt und der Kolonne vollständig zugeführt. Es wurde weiterhin vom Phasenscheider 135 g/h dem ersten Rührkessel der Rührkesselkaskade der Aldolreaktion zugeführt. Um die Regeltemperatur in der Kolonne auf 85°C halten, wurden zusätzlich der Kolonne 25 g/h organische Phase zugeführt. In der dem Kondensator nachgeschalteten Kühlfalle fielen ca. 1 g/h Flüssigkeit an (ca. 80 Gew.-% (Isobutyraldehyd (IBA), ca. 20 Gew.-% TMA), die ebenfalls rückgeführt wurden.
Die IBA-Abtrennung wurde bei einem Kopfdruck von ca. 1 bar absolut betrieben. Als Verdampfer wurde ein Fallfilmverdampfer verwendet. Es wurde eine Sumpftemperatur im Sumpf der Kolonne von 104°C eingestellt. Die Rücklaufmenge (bzw. Kühlwassermenge des Partialkondensators) zur Kolonne wurde mittels der Temperatur in der Mitte der Gewebepackung geregelt, es wurde eine Temperatur von 85°C eingestellt.
Aus dem Kolonnensumpf wurde mittels einer Pumpe ca. 100 kg/h Flüssigkeit abgezogen. Diese wurde dem Fallfilmverdampfer (bestehend aus einem ölbeheizten Edelstahlrohr, Länge, 2,5 m, Innendurchmesser ca. 21 mm, Wandstärke ca. 2mm) zugeführt. Es wurde aus dem Sumpf des Fallfilmverdampfers ca. 1,5 kg/h Produkt mit einer Konzentration von ca. 0,3 Gew.-% Isobutyraldehyd abgezogen. Die Brüden und überschüssige Flüssigkeit wurden dem Kolonnensumpf zugeführt. Das ausgeschleuste Sumpfprodukt enthielt ca. 70 Gew.-% HPA, ca. 1,5 Gew.-% HPN, 0,3 Gew.-% Isobutyraldehyd (IBA), Rest Wasser.

### Stufe c) Hydrierung des Sumpfaustrags aus Stufe b):

Das erhaltene Sumpfprodukt wurde anschließend einer Hydrierung mittels Festbett unterzogen.
Die Aktivierung des Katalysators erfolgte folgendermaßen:
150 ml eines Cu/Al₂O₃-Katalysators wie in der EP 44444 beschrieben wurden in einem Rohrreaktor bei 190°C durch Überleiten eines Gemisches aus 5 Vol.-% Wasserstoff und 95 Vol.-% Stickstoff (Gesamtvolumen 50 Nl/h) drucklos 24 Stunden aktiviert.
Die Hydrierung wurde wie folgt ausgeführt:
Als Ausgangslösung diente das vorstehend als Hydrierfeed beschriebene Gemisch. Dem Gemisch wurden ca. 10 Gew.-% bezogen auf den Hydrierfeed einer 15 Gew.-%igen wässrigen Lösung von Trimethylamin zugesetzt. Der so erhaltene Zulauf wurde in Rieselfahrweise bei 40 bar H₂-Druck über den auf 120°C beheizten Reaktor gefahren. Die Belastung betrug 0,4 kg HPA/ (I_{Kat.}*h). Ein Teil des Hydrieraustrags wurde dem Zulauf wieder zugemischt (Kreislauffahrweise). Das Verhältnis von Kreislauf zu Zulauf betrug 10:1. Der pH-Wert wurde von Proben des Reaktoraustrags bei Raumtemperatur zu 8,9 gemessen.
Die Zusammensetzung des wässrigen Polymethylolgemisches aus Stufe c) betrug:
   NPG: 69 Gew.-%
   Methanol: 3,5 Gew.-%
   TMA: 2 Gew.-%
   organische Nebenverbindungen (HPS, iso-Butanol): <2 Gew.-%
   TMA-Formiat: 1 Gew.%
   Wasser: 23 Gew.-%

### Stufe d): Destillation des wässrigen Polymethylolgemisches aus Stufe c):

Der erhalten Austrag (ca. 1,5 kg/h) wurde einer destillativen Trennung zugeführt. Es wird eine Packungskolonne, (DN 50 mm) mit drei Sequenzen von strukturierter Blech-Packung mit je 1 m Länge und 500 m2/m3 spezifischer Oberfläche verwendet. Der Zulauf erfolgte oberhalb der untersten Sequenz. Es wurde ein Kopfdruck von. ca. 175 mbar absolut eingestellt. Im Sumpf wurde eine Temperatur von 160 bis 165°C eingestellt, die Energie wurde der Kolonne mittels eines Naturumlaufverdampfers zugeführt. Die am Kopf erhaltenen Brüden wurden einem Kondensator zugeführt, dieser schlägt bei 30°C die erhaltenen Brüden nahezu vollständig nieder. Das Vakuum wurde mittels einer einfachen handelsüblichen Wasserstrahlvakuumpumpe erzeugt. Vom erhaltenen Destillat ("Leichtsiedergemisch") wurden ca. 350 g/h ausgeschleust, ca. 250 g/h wurden der Kolonne auf dem obersten Packungsabschnitt als Rücklauf zudosiert. Das zur Vakuumerzeugung verwendete Wasser wurde einer biologischen Abwasserbehandlung zugeführt.

Das Leichtsiedergemisch aus Stufe d) wies folgende Zusammensetzung auf:
11 Gew.-% Methanol,
0,1 Gew.-% nicht umgesetzter Aldehyd,
0,5 Gew.-% ,NPG,
1,5 Gew.-% tert. Amin,
1 % TMA-Formiat
0,1 Gew.-% Methylolalkanal (II),
4 %Isobutanol, Alkohol (III),
Rest organische Nebenverbindungen,
ca. 80 % Rest Wasser.

### Beispiel 1: Destillation des Leichtsiedergemischs

Das Leichtsiedergemisch aus Stufe d) wurde gesammelt, mit 0,02 g wässriger NaOH (25%) pro Gramm Leichtsiedergemisch versetzt und einer destillativen Trennung zugeführt ("Leichtsiederdestillation").
Es wurde eine Packungskolonne, (DN 50 mm) mit drei Schüssen von strukturierter Packung mit je 1 m Länge und 500 m²/m³ spezifischer Oberfläche verwendet. Der Zulauf (ca. 4 kg/h) erfolgte oberhalb des zweiten Abschnitts. Es wurde ein Kopfdruck von. ca. 1,05 bar absolut eingestellt. Im Sumpf wurde eine Temperatur von 104°C eingestellt. Die Energie wurde der Kolonne mittels eines Naturumlaufverdampfers zugeführt. Die am Kopf erhaltenen Brüden wurden einem Kondensator zugeführt, dieser schlug bei 30°C die erhaltenen Brüden nahezu vollständig nieder. Vom erhaltenen Destillat wurden ca. 1500 g/h ausgeschleust, ca. 1000 g/h wurden der Kolonne auf dem obersten Packungsabschnitt als Rücklauf zudosiert.

Die erhaltenen organischen Leichtsieder wurden gesammelt und zu 1,5 kg/h einer Bodenkolonne (Durchmesser DN 50 mm, 40 Böden) zugeführt ("TMA-Destillation").

Der Zulauf erfolgte auf dem 30. Boden von unten. Die Kolonne wurde bei 5 bar Überdruck betrieben. Im Sumpf wurde die Kolonne mittels eines Naturumlaufverdampfers aufgekocht. Es wurde eine Temperatur 130°C (gemessen im 5. Boden der Kolonne) eingestellt. Am Kopf der Kolonne wurden die Brüden in einem Kondensator bei 30°C nahezu vollständig niedergeschlagen. Von dem erhaltenen Kondensat wurden ca. 140 g/h ausgeschleust, zur Kolonne wurden 1,25 kg/h als Rücklauf auf den obersten Boden rückgeführt. Aus dem Kolonnensumpf wurden ca. 1,35 kg/h (ca. 45% Wasser, ca. 40 % MeOH, ca. 15% IBOL (iso-Butanol), < 100 Gew.-ppm TMA, Rest sonstige organische Komponenten) ausgeschleust.
Das ausgeschleuste Kondensat enthielt > 99% TMA (Rest überwiegend Methanol). Die Verweilzeit betrug ca. 11 Minuten (V_{Hold-up}= 30 x 20 ml = 0,6 I; V_{Zulauf}= 1,5 kg/h / 0,8 kg/l = 1,88 l/h, V_{Rücklauf} = 1,25 kg/h / 0,8 kg/l = 1,56 l/h).

Das aus der TMA-Abtrennung erhaltene Sumpfprodukt wurde gesammelt und einer weiteren destillativen Trennung zugeführt ("MeOH-Destillation). Es wurde eine Packungskolonne (DN 50 mm) mit drei Sequenzen von strukturierter Packung mit je 1 m Länge und 500 m²/m³ spezifischer Oberfläche verwendet. Der Zulauf (ca. 1,3 kg/h) erfolgte oberhalb des zweiten Abschnitts. Es wurde ein Kopfdruck von. ca. 1,05 bar absolut eingestellt. Im Sumpf wurde eine Temperatur von 92°C eingestellt, die Energie wurde der Kolonne mittels eines Naturumlaufverdampfers zugeführt. Die am Kopf erhaltenen Brüden wurden einem Kondensator zugeführt, welcher die erhaltenen Brüden bei 30°C nahezu vollständig niederschlägt. Vom erhaltenen Destillat (99,86% Methanol, Rest überwiegend Wasser), wurden ca. 500 g/h ausgeschleust, ca. 2000 g/h wurden der Kolonne auf dem obersten Packungsabschnitt als Rücklauf zudosiert.
Der im Sumpf der Kolonne ausgeschleuste Sumpf wurde in einem Wärmetauscher auf ca. 30°C abgekühlt und einem Phasenscheider gesammelt. Anschließend wurde eine organische Phase (überwiegend Isobutanol, ca. 10% Wasser, ca. 1% MeOH, Rest sonstige organische Komponenten, 200 g/h bei 1,3 kg/h Feed) und eine wässrige Phase (ca. 90 oder auch mehr % Wasser, ca. 5% Isobutanol, ca. 2% Methanol, Rest sonstige organische Komponenten, 600 g/h bei 1,3 kg/h Feed) erhalten.

### Beispiel 2: Destillation des Leichtsiedergemischs in einer Trennwandkolonne

Das Leichtsiedergemisch aus Stufe d) wies die gleiche Zusammensetzung, wie in Beispiel 1 auf. Das aus Kopfprodukt aus Stufe d) wurde gesammelt, mit 0,02 g wässrige NaOH (25%) pro Gramm Leichtsiedergemisch versetzt und einer destillativen Trennung zugeführt ("Leichtsieder-Destillation"). Die "Leichtsiederdestillation" erfolgte analog zu der "Leichtsieder-Destillation" in Beispiel 1.

Die Aufarbeitung der aus der "Leichtsieder-Destillation" erhaltenen organischen Leichtsieder erfolgte in einer Trennwandkolonne.

Die erhaltenen organischen Leichtsieder aus der Leichtsiederdestillation wurden gesammelt und mengengeregelt auf 1 kg/h einer Trennwandkolonne (Bodenkolonne Durchmesser DN 80 mm, 60 Böden, Trennblech vom 16 bis 45 Boden) zugeführt, der Zulauf erfolgt auf dem 30. Boden, bzw. dem 15 Boden auf der Zulaufseite des durch das Trennblech unterteilten Segments. Die Kolonne wurde bei 2 bar Überdruck betrieben.

Im Sumpf wurde die Kolonne mittels eines Naturumlaufverdampfers aufgekocht. Es wurde eine Temperatur von 125°C eingestellt. Am Kopf der Kolonne wurden die Brüden in einem Kondensator bei 30°C nahezu vollständig niedergeschlagen. Von dem erhaltenen Kondensat (> 99% TMA, Rest überwiegend Methanol) wurden ca. 100 g/h ausgeschleust, zur Kolonne wurden 4 kg/h als Rücklauf auf den obersten Boden rückgeführt.
In der Kolonne wurde oberhalb des Trennblechabschnittes die Flüssigkeit vollständig gesammelt. Es wurden ca. 1,75 kg Flüssigkeit erhalten. Diese 1,75 kg wurden zu ca. 500 g/h dem Zulaufabschnitt zugeführt, 1250 g/h wurden mengengeregelt der Produktseite zugeführt. Auf dem 30. Boden, bzw. 15. Boden des Trennblechabschnitts wurden 350 g/h methanolreiche Flüssigkeit (>99,9% Methanol, Rest überwiegend Wasser) der Kolonne entnommen.
Aus dem Kolonnensumpf wurden ca. 550 g/h ausgeschleust. Der im Sumpf der Kolonne ausgeschleuste Sumpf wurde in einem Wärmetauscher auf ca. 30°C abgekühlt und in einem Phasenscheider gesammelt. Anschließend wurde eine organische Phase (überwiegend Isobutanol, ca. 10% Wasser, ca. 0,5 % MeOH, Rest sonstige organische Komponenten) und eine wässrige Phase (ca. 90 oder auch mehr % Wasser, ca. 5% Isobutanol, ca. 1 % Methanol, Rest sonstige organische Komponenten) erhalten.
Die wässrige Phase wurde ausgeschleust. Die organische Phase wurde ebenfalls ausgeschleust.
Die Verweilzeit in der Trennwandkolonne betrug ca. 7 Minuten (V_{Hold-up} = 15 x 0,05 l = 0,75 l; V_{Zulauf}=1 kg/h/0,8 kg/l =1,25 l/h; V_{Rücklauf} = 4 kg/h/0,8 kg/l = 5 l/h)

### Beispiel 3: Vergleichsbeispiel (Destillation des Leichtsiedergemischs)

Das Leichtsiedergemisch aus Stufe d) wies die gleiche Zusammensetzung, wie in Beispiel 1 auf. Das aus Kopfprodukt aus Stufe d) wurde gesammelt mit 0,02 g/g Destillat wässrige NaOH (25%) gemischt und einer destillativen Trennung zugeführt ("Leichtsieder-Destillation").
Die Zuführung erfolgte mit 1,5 kg/h in eine Bodenkolonne (Durchmesser DN 50 mm, 40 Böden). Der Zulauf erfolgte auf dem 30. Boden von unten. Die Kolonne wurde bei 1 bar Überdruck betrieben. Im Sumpf wurde die Kolonne mittels eines Naturumlaufverdampfers (der Verdampfer kann aber auch eine andere Art an Verdampfer, z.B. Fallfilmverdampfer sein) aufgekocht. Es wurde eine Temperatur von 100°C (gemessen auf dem 5. Boden) eingestellt. Die Sumpftemperatur lag somit unter 110°C. Am Kopf der Kolonne wurden die Brüden in einem Kondensator bei 5°C nahezu vollständig niedergeschlagen. Von dem erhaltenen Kondensat wurden ca. 150 g/h ausgeschleust, zur Kolonne wurden 5 kg/h als Rücklauf auf den obersten Boden rückgeführt. Aus dem Kolonnensumpf wurden ca. 1,3 kg/h (ca. 45% Wasser, ca. 39 % MeOH, ca. 15% Isobutanol, ca. 1% Gew.-% TMA, Rest sonstige organische Komponenten) ausgeschleust.

Die Verweilzeit im Abtriebsteil betrug ca. 4 Minuten (V_{Hold-up} = 30 x 0,02 l = 0,6 I; V_{Zulauf}=1,5 kg/h/0,8 kg/l =1,88 l/h; V_{Rücklauf} = 5 kg/h/0,8 kg/l = 6,25 l/h)

Das ausgeschleuste Kondensat enthielt ca. 95% TMA, ca. 1% Wasser, ca. 4% MeOH.

Der aus der TMA-Abtrennung erhaltene Rückstand wurde gesammelt und einer weiteren destillativen Trennung zugeführt ("MeO-Destillation). Es wurde eine Packungskolonne (DN 50 mm) mit drei Sequenzen von strukturierter Packung mit je 1 m Länge und 500 m2/m3 spezifischer Oberfläche verwendet. Der Zulauf (ca. 1,3 kg/h) erfolgte oberhalb des zweiten Abschnitts. Es wurde ein Kopfdruck von. ca. 1,05 bar absolut eingestellt. Im Sumpf wurde eine Temperatur von 92°C eingestellt, die Energie wurde der Kolonne mittels eines Naturumlaufverdampfers zugeführt, es kann aber auch ein anderer Verdampfer, z.B. Fallfilmverdampfer eingesetzt werden. Die am Kopf erhaltenen Brüden wurden einem Kondensator zugeführt, dieser schlägt bei 30°C die erhaltenen Brüden nahezu vollständig nieder. Vom erhaltenen Destillat (ca. 98% Methanol, ca. 1 % IBA, ca. 1% TMA), wurden ca. 550 g/h ausgeschleust, ca. 2000 g/h wurden der Kolonne auf dem obersten Packungsabschnitt als Rücklauf zudosiert.

Der Vergleich von Beispiel 1 mit Beispiel 3 (Vergleichsbeispiel) zeigt, dass das Kondensat der zweiten Destillationsstufe ("TMA-Destillation") nur dann einen geringen Methanolgehalt aufweist, wenn die Sumpftemperatur mehr als 110°C beträgt. Wird die Destillation bei niedrigeren Temperaturen durchgeführt, wird TMA erhalten, dass noch größere Mengen MeOH enthält. Ein solches methanolhaltiges TMA ist für die Rückführung in die Aldolreaktion weniger gut geeignet, als das TMA, welches durch das erfindungsgemäße Verfahren erhalten wurde.

### Beispiel 4:

### Herstellung von Roh-Polymethylol mit dem Hydrierverfahren mit TEA

### Stufe a) Aldolreaktion:

Es wurde ca. 750 g/h Isobutyraldehyd (ca. >99,5 GC-Flächen-% IBA) mit ca. 700 g/h Formaldehyd (ca. 49 Gew.-% Formaldehyd, 1,5 Gew.-% Methanol, Rest Wasser) und 30 g/h Triethylaminlösung in einer zweistufigen Rührkesselkaskade zur Reaktion gebracht.

### Stufe b) Destillative Auftrennung des Reaktionsgemisches aus Stufe a):

Anschließend wurde die Lösung in einer Kolonne destillativ von Leichtsiedern befreit. Die Kolonne war mit 1,5 m Gewebepackung (500m²/m³ spezifische Oberfläche) im Verstärkungsteil und 4 m Blechpackung (250 m²/m³) ausgerüstet. Der Aldolisierungsaustrag wurde oberhalb der Blechpackung zugeführt. Am Kopf der Kolonne wurde ein Kondensator mit Kühlwasser (ca. 10°C) und einem nachgeschalteten Phasenscheider verwendet. Am Kopf wurde das Destillat gasförmig dem Kondensator zugeführt. Es fielen ca. 227 g/h flüssiges Kondensat an. In dem nachgeschalteten Phasenscheider wurde eine wässrige Phase von 55 g/h abgetrennt und der Kolonne vollständig zugeführt. Es wurde weiterhin vom Phasenscheider 80 g/h dem ersten Rührkessel der Rührkesselkaskade der Aldolreaktion zugeführt. Um die Regeltemperatur in der Kolonne auf 85°C halten, wurden zusätzlich der Kolonne ca. 92 g/h organische Phase zugeführt. In der dem Kondensator nachgeschalteten Kühlfalle fielen ca. 1 g/h Flüssigkeit an (ca. 90 Gew.-% IBA, ca. 1 Gew.-% TEA, Rest weitere org. Komp.), die ebenfalls rückgeführt wurden.
Die IBA-Abtrennung wurde bei einem Kopfdruck von ca. 1 bar absolut betrieben. Als Verdampfer wurde ein Fallfilmverdampfer verwendet. Es wurde eine Sumpftemperatur im Sumpf der Kolonne von 103°C eingestellt. Die Rücklaufmenge (bzw. Kühlwassermenge des Partialkondensators) zur Kolonne wurde mittels der Temperatur in der Mitte der Gewebepackung geregelt. Es wurde eine Temperatur von 85°C eingestellt.
Aus dem Kolonnensumpf wurde mittels einer Pumpe ca. 100 kg/h Flüssigkeit abgezogen. Diese wurde dem Fallfilmverdampfer (bestehend aus einem Ölbeheizten Edelstahlrohr, Länge, 2,5 m, Innendurchmesser ca. 21 mm, Wandstärke ca. 2mm) zugeführt. Es wurde aus dem Sumpf des Fallfilmverdampfers ca. 1,5 kg/h Produkt mit einer Konzentration von ca. 0,3 Gew.-% Isobutyraldehyd abgezogen. Die Brüden und überschüssige Flüssigkeit wurden dem Kolonnensumpf zugeführt. Das ausgeschleuste Sumpfprodukt enthielt ca. 70 Gew.-% Hydroxypivalinaldehyd (HPA), ca. 1% TEA 1-2% TEA-Formiat, ca. 1 Gew.-% Hydroxypivalinsäureneopentylglykolester (HPN), 0,6 Gew.-% IBA, Rest Wasser.

### Stufe c) Hydrierung des Sumpfaustrags aus Stufe b):

Das erhaltene Sumpfprodukt wurde anschließend einer Hydrierung mittels Festbett unterzogen.
Die Aktivierung des Katalysators erfolgte folgendermaßen:
150 ml eines Cu/Al₂O₃-Katalysators, wie in der EP 44444 beschrieben, wurden in einem Rohrreaktor bei 190°C durch Überleiten eines Gemisches aus 5 Vol.-% Wasserstoff und 95 Vol.-% Stickstoff (Gesamtvolumen 50 Nl/h) drucklos 24 Stunden aktiviert. Die Hydrierung wurde wie folgt ausgeführt:
   Als Ausgangslösung diente das vorstehend als Hydrierfeed beschriebene Gemisch. Der Zulauf wurde in Rieselfahrweise bei 40 bar H₂-Druck über den auf 120°C beheizten Reaktor gefahren. Die Belastung betrug 0,4 kg HPA/ (I_{Kat.}*h). Ein Teil des Hydrieraustrags wurde dem Zulauf wieder zugemischt (Kreislauffahrweise). Das Verhältnis von Kreislauf zu Zulauf betrug 10:1. Der pH-Wert wurde von Proben des Reaktoraustrags bei Raumtemperatur zu 8 gemessen.
   Die Zusammensetzung des wässrigen Polymethylolgemisches aus Stufe c) betrug:
      NPG: 69 Gew.-%
      Methanol: 3,5 Gew.-%
      TEA: 1 Gew.-%
      organische Nebenverbindungen (HPS, n-Butanol): <2 Gew.-%
      TEA-Formiat: ca. 1 Gew.%
      Wasser: 23 Gew.-%

### Stufe d): Destillation des wässrigen Polymethylolgemisches aus Stufe c):

Der erhaltene Austrag wird gesammelt und zu ca. 1,5 kg/h einer destillativen Trennung zugeführt. Es wird eine Packungskolonne, (DN 50 mm) mit drei Sequenzen von strukturierter Blech-Packung mit je 1 m Länge und 500 m²/m³ spezifischer Oberfläche verwendet. Der Zulauf erfolgte oberhalb der untersten Sequenz. Es wurde ein Kopfdruck von. ca. 175 mbar absolut eingestellt. Im Sumpf wurde eine Temperatur von 160 bis 165°C eingestellt, die Energie wurde der Kolonne mittels eines Naturumlaufverdampfers zugeführt. Die am Kopf erhaltenen Brüden wurden einem Kondensator zugeführt, dieser schlägt bei 30°C die erhaltenen Brüden nahezu vollständig nieder. Das Vakuum wurde über einer einfachen handelsüblichen Wasserstrahlvakuumpumpe erzeugt. Vom erhaltenen Destillat ("Leichtsiedergemisch") wurden ca. 350 g/h ausgeschleust, ca. 250 g/h wurden der Kolonne auf dem obersten Packungsabschnitt als Rücklauf zudosiert. Das zur Vakuumerzeugung verwendete Wasser wurde einer biologischen Abwasserbehandlung zugeführt.

Das Leichtsiedergemisch aus Stufe d) hatte folgende Zusammensetzung:
10 Gew.-% Methanol,
0,1 Gew.-% nicht umgesetzter Aldehyd,
0,5 Gew.-% Alkohol (III, NPG),
1 Gew.-% tert. Amin,
1,5 % TEA-Formiat
0,2 Gew.-% Methylolalkanal (II),
4% IBuOH
ca. 1 Gew.% organische Nebenverbindungen,
Rest Wasser.

### Destillation des Leichtsiedergemischs

Das aus Kopfprodukt aus Stufe d) wurde gesammelt, mit 0,02 g wässriger NaOH (25%) pro Gramm Leichtsiedergemisch versetzt und einer destillativen Trennung zugeführt ("Leichtsieder-Destillation"). Es wurde eine Packungskolonne, (DN 50 mm) mit drei Schüssen von strukturierter Packung mit je 1 m Länge und 500 m2/m3 spezifischer Oberfläche verwendet. Der Zulauf (ca. 4 kg/h) erfolgte oberhalb des zweiten Abschnitts. Es wurde ein Kopfdruck von. ca. 1,05 bar absolut eingestellt. Im Sumpf wurde eine Temperatur von ca. 103°C eingestellt, die Energie wurde der Kolonne mittels eines Naturumlaufverdampfers zugeführt (es kann aber auch ein anderer Verdampfer, z.B. Fallfilmverdampfer eingesetzt werden). Die am Kopf erhaltenen Brüden wurden einem Kondensator zugeführt, dieser schlug bei 30°C die erhaltenen Brüden nahezu vollständig nieder. Vom erhaltenen Destillat wurden ca. 1400 g/h ausgeschleust, ca. 800 g/h wurden der Kolonne auf dem obersten Packungsabschnitt als Rücklauf zudosiert.

Das aus Kopfprodukt aus der ersten Stufe der mehrstufigen Leichtsiederdestillation Stufe wurde gesammelt, und einer destillativen Trennung, der sogenannten "TEA-Destillation" zugeführt.
Die Zuführung von 1 kg/h erfolgte in eine Bodenkolonne (Durchmesser DN 50 mm, 40 Böden). Der Zulauf erfolgte auf dem 30. Boden von unten. Die Kolonne wurde bei 2,5 bar Überdruck betrieben. Im Sumpf wurde die Kolonne mittels eines Naturumlaufverdampfers aufgekocht. Es wurde eine Temperatur von 125°C eingestellt (gemessen im 5. Boden der Kolonne). Am Kopf der Kolonne wurden die Brüden in einem Kondensator bei 30°C nahezu vollständig niedergeschlagen. Von dem erhaltenen Kondensat (> 29% TEA, ca. 3 % Wasser, 67% Methanol) wurden ca. 400g/h ausgeschleust, zur Kolonne wurden 2,0 kg/h als Rücklauf auf den obersten Boden rückgeführt. Aus dem Kolonnensumpf wurden ca. 0,6 kg/h (ca. 73% Wasser, ca. 1 % MeOH, ca. 24% iso-Butanol, < 100 Gew.-ppm TEA, Rest sonstige organische Komponenten) ausgeschleust.
Die Verweilzeit im Abtriebsteil betrug ca. 10 Minuten (V_{Hold-up} = 30 x 0,02 l = 0,6 I; V_{Zulauf}=1 kg/h/0,8 kg/l =1,25 l/h; V_{Rücklauf} = 2 kg/h/0,8 kg/l = 2,5 l/h).

Der Sumpfrückstand wurde auf ca. 20°C abgekühlt, es bildeten sich zwei Phasen, die organische (ca. 20% Massenanteil, ca. 78% Isobutanol, ca. 18% Wasser, 1% MeOH) und eine wässrige (ca. 80%, ca. 84% Wasser, ca. 15% Isobutanol, ca. 1% MEOH).

Das aus der TEA-Abtrennung erhaltene Destillat wurde gesammelt und einer weiteren destillativen Trennung zugeführt ("MeOH-Destillation"). Es wurde eine Packungskolonne (DN 50 mm) mit drei Sequenzen von strukturierter Packung mit je 1 m Länge und 500 m²/m³ spezifischer Oberfläche verwendet. Der Zulauf (ca. 0,5 kg/h) erfolgt oberhalb des zweiten Abschnitts. Es wurde ein Kopfdruck von. ca. 1,05 bar absolut eingestellt. Im Sumpf wurde eine Temperatur von 75°C eingestellt, die Energie wurde der Kolonne mittels eines Naturumlaufverdampfers zugeführt, es kann aber auch ein anderer Verdampfer, z.B. Fallfilmverdampfer eingesetzt werden. Die am Kopf erhaltenen Brüden wurden einem Kondensator zugeführt, dieser schlägt bei 30°C die erhaltenen Brüden nahezu vollständig nieder. Vom erhaltenen Destillat (99,86% Methanol, Rest überwiegend Wasser und TEA), wurden ca. 340 g/h ausgeschleust, ca. 4,5 kg/h wurden der Kolonne auf dem obersten Packungsabschnitt als Rücklauf zudosiert.

Der im Sumpf der Kolonne ausgeschleuste Sumpf wurde in einem Wärmetauscher auf ca. 20°C abgekühlt und gesammelt (ca. 165 g/h, ca. 85 TEA, ca. 10% Wasser, ca. 2% MEOH). Nach längerer Verweilzeit bildeten sich zwei Phasen, die organische (ca. 150 g/h, 92% TEA,) wurde gesammelt und in der Aldolreaktion eingesetzt.
Die wässrige Phase wurde verworfen.

## Patentansprüche

1. Verfahren zur Rückgewinnung von Komponenten aus einem Leichtsiedergemisch, das bei der Destillation von Hydrierausträgen aus der Herstellung von Polymethylolen anfällt, durch mehrstufige Destillation des Leichtsiedergemischs, enthaltend ein tertiäres Amin, Wasser, Methanol, Polymethylol der Formel (I), 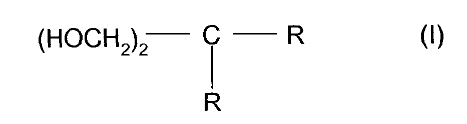 Methylolalkanal der Formel (II), 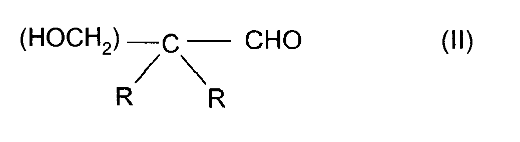 Alkohol der Formel (III)), 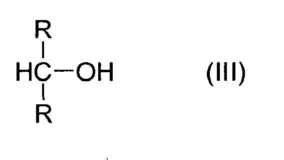 und ein Alkanal mit einer Methylengruppe in α-Stellung zur Carbonylgruppe,
und in der R jeweils unabhängig voneinander eine weitere Methylolgruppe oder eine Alkylgruppe mit 1 bis 22 C-Atomen oder eine Aryl- oder Aralkylgruppe mit 6 bis 22 C-Atomen bedeutet,
wobei in einer ersten Destillationsstufe eine Auftrennung des Leichtsiedergemischs in eine höhersiedende, überwiegend wasserreiche Fraktion und in eine das tertiäre Amin enthaltende leichtersiedende wässrige, organische Fraktion erfolgt,
und in der zweiten Destillationsstufe die wässrige, organische Fraktion aus der ersten Destillationsstufe in eine überwiegend aminhaltige Fraktion und eine weitere aminabgereicherte Fraktion aufgetrennt wird,
**dadurch gekennzeichnet, dass** das tertiäre Amin Trimethylamin oder Triethylamin ist und die Sumpftemperatur in der zweiten Destillationsstufe 110°C und mehr beträgt.

2. Verfahren nach Anspruch 1, **dadurch gekennzeichnet, dass** man dem Leichtsiedergemisch vor Einführung in die erste Destillationsstufe eine Base zusetzt.

3. Verfahren nach mindestens einem der Ansprüche 1 bis 2, **dadurch gekennzeichnet, dass** das Methylolalkanal der Formel (I) Neopentylglykol, Trimethylolpropan, Pentaerythrit, Trimethylolethan oder Trimethylolbutan ist.

4. Verfahren nach mindestens einem der Ansprüche 1 bis 3, **dadurch gekennzeichnet, dass** das tertiäre Amin Trimethylamin ist.

5. Verfahren nach mindestens einem der Ansprüche 1 bis 4, **dadurch gekennzeichnet, dass** das Leichtsiedergemisch
1 bis 20 Gew.-% Methanol,
0,01 bis 1% nicht umgesetzter Aldehyd,
0,5 bis 5 Gew.-% Alkohol (III),
0,5 bis 5 Gew.-% tert. Amin,
0,01 bis 1 Gew.-% Methylolalkanal (II),
0 bis 5 Gew.% organische Nebenverbindungen,
Rest Wasser, enthält.

6. Verfahren nach mindestens einem der Ansprüche 1 bis 5, **dadurch gekennzeichnet, dass** die Sumpftemperatur in der zweiten Destillationsstufe im Bereich von 110 bis 145°C liegt.

7. Verfahren nach mindestens einem der Ansprüche 1 bis 6, **dadurch gekennzeichnet, dass** Verweilzeit in der zweiten Destillationsstufe im Bereich von 5 bis 45 Minuten beträgt.

8. Verfahren nach mindestens einem der Ansprüche 1 bis 7, **dadurch gekennzeichnet, dass** der Druck in der zweiten Destillationsstufe im Bereich von 3 bis 10 bar liegt.

9. Verfahren nach mindestens einem der Ansprüche 1 bis 8, **dadurch gekennzeichnet, dass** die überwiegend aminhaltige Fraktion aufgearbeitet wird und als Katalysator für die Umsetzung von Alkanalen mit Formaldehyd eingesetzt wird.

10. Verfahren nach mindestens einem der Ansprüche 1 bis 9, **dadurch gekennzeichnet, dass** das eingesetzte Amin Triethylamin ist und die überwiegend a-minhaltige Fraktion aus der zweiten Destillationsstufe in einer dritten Destillationsstufe aufgetrennt wird in eine Fraktion, die überwiegend tertiäres Amin und in eine Fraktion, die überwiegend Wasser bzw. Methanol enthält.

11. Verfahren nach mindestens einem der Ansprüche 1 bis 9, **dadurch gekennzeichnet, dass** das eingesetzt Amin Trimethylamin ist und die wässrig, organische Fraktion aus der zweiten Destillationsstufe in eine methanolische Phase und eine wässrig, organische Fraktion aufgetrennt wird.

12. Verfahren nach Anspruch 10 oder Anspruch 11, **dadurch gekennzeichnet, dass** die zweite und die dritte Destillationsstufe gemeinsam in einer Trennwandkolonne durchgeführt werden.

13. Verfahren nach Anspruch 10 oder Anspruch 12, **dadurch gekennzeichnet, dass** das Triethylamin aus der dritten Destillationsstufe als Katalysator für die Umsetzung von Alkanalen mit Formaldehyd eingesetzt wird.

14. Verfahren nach Anspruch 11 oder Anspruch 12, **dadurch gekennzeichnet, dass** das Trimethylamin aus der zweiten Destillationsstufe als Katalysator für die Umsetzung von Alkanalen mit Formaldehyd eingesetzt wird.

15. Verfahren nach mindestens einem der Ansprüche 1 bis 14, **dadurch gekennzeichnet, dass** man das Leichtsiedergemisch durch ein mehrstufiges Hydrierverfahren erhält, wobei man in Stufe a) Alkanale mit Formaldehyd in Gegenwart von tertiären Aminen als Katalysator zu Methylolalkanalen der Formel (II) kondensiert, und man anschließend in Stufe b) das aus Stufe a) erhaltenen Reaktionsgemisch in einen Sumpf, der überwiegend Verbindungen der Formel (II) enthält, und einen Kopfstrom, der Leichtsieder enthält, destillativ auftrennt, und man in Stufe c) den Sumpfaustrag aus Stufe b) hydriert und man anschließend in einer Stufe d) den Austrag aus Stufe c) destilliert, wobei das Leichtsiedergemisch aus Stufe d) abgetrennt wird.

## Claims

1. A process for recovering components from a low boiler mixture which is obtained in the distillation of hydrogenation effluents from the preparation of polymethylols, by multistage distillation of the low boiler mixture comprising a tertiary amine, water, methanol, polymethylol of the formula (I) 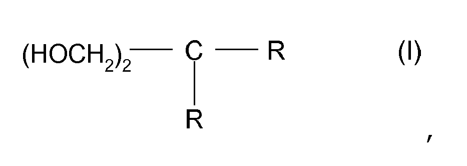 methylolalkanal of the formula (II) 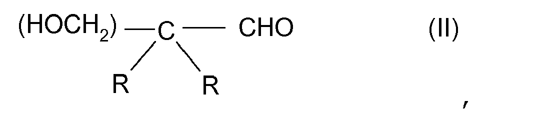 alcohol of the formula (III) 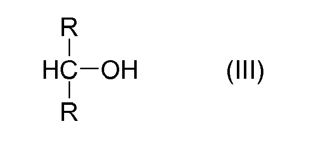 and an alkanal with a methylene group in the α position to the carbonyl group,
and in which each R is independently a further methylol group or an alkyl group having 1 to 22 carbon atoms or an aryl or aralkyl group having 6 to 22 carbon atoms,
a first distillation stage involving separating the low boiler mixture into a higher-boiling, predominantly water-rich fraction and into a lower-boiling aqueous organic fraction comprising the tertiary amine,
and the second distillation stage involving separating the aqueous organic fraction from the first distillation stage into a predominantly amine-containing fraction and a further amine-depleted fraction,
wherein the tertiary amine is trimethylamine or triethylamine, and the bottom temperature in the second distillation stage is 110°C and more.

2. The process according to claim 1, wherein a base is added to the low boiler mixture before introduction into the first distillation stage.

3. The process according to at least one of claims 1 and 2, wherein the methylolalkanal of the formula (I) is neopentyl glycol, trimethylolpropane, pentaerythritol, trimethylolethane or trimethylolbutane.

4. The process according to at least one of claims 1 to 3, wherein the tertiary amine is trimethylamine.

5. The process according to at least one of claims 1 to 4, wherein the low boiler mixture comprises 1 to 20% by weight of methanol,
0.01 to 1% unconverted aldehyde,
0.5 to 5% by weight of alcohol (III),
0.5 to 5% by weight of tertiary amine,
0.01 to 1% by weight of methylolalkanal (II),
0 to 5% by weight of organic secondary compounds,
remainder water.

6. The process according to at least one of claims 1 to 5, wherein the bottom temperature in the second distillation stage is in the range from 110 to 145°C.

7. The process according to at least one of claims 1 to 6, wherein the residence time in the second distillation stage is in the range from 5 to 45 minutes.

8. The process according to at least one of claims 1 to 7, wherein the pressure in the second distillation stage is in the range from 3 to 10 bar.

9. The process according to at least one of claims 1 to 8, wherein the predominantly amine-containing fraction is worked up and used as a catalyst for the reaction of alkanals with formaldehyde.

10. The process according to at least one of claims 1 to 9, wherein the amine used is triethylamine and the predominantly amine-containing fraction from the second distillation stage is separated in a third distillation stage into a fraction which comprises predominantly tertiary amine and into a fraction which comprises predominantly water and/or methanol.

11. The process according to at least one of claims 1 to 9, wherein the amine used is trimethylamine and the aqueous organic fraction from the second distillation stage is separated into a methanolic phase and an aqueous organic fraction.

12. The process according to claim 10 or claim 11, wherein the second and the third distillation stages are performed together in one dividing wall column.

13. The process according to claim 10 or claim 12, wherein the triethylamine from the third distillation stage is used as a catalyst for the reaction of alkanals with formaldehyde.

14. The process according to claim 11 or claim 12, wherein the trimethylamine from the second distillation stage is used as a catalyst for the reaction of alkanals with formaldehyde.

15. The process according to at least one of claims 1 to 14, wherein the low boiler mixture is obtained by a multistage hydrogenation process involving, in stage a), condensing alkanals with formaldehyde in the presence of tertiary amines as a catalyst to give methylolalkanals of the formula (II), and then, in stage b), distillatively separating the reaction mixture obtained from stage a) into bottoms comprising predominantly compounds of the formula (II), and a topstream comprising low boilers, and, in stage c), hydrogenating the bottom effluent from stage b) and then, in a stage d), distilling the effluent from stage c), the low boiler mixture being removed from stage d).

## Revendications

1. Procédé de récupération de composants à partir d'un mélange de composants de point d'ébullition faible, qui se forme lors de la distillation de sorties d'hydrogénation issues de la fabrication de polyméthylols, par distillation à plusieurs étapes du mélange de composants de point d'ébullition faible contenant une amine tertiaire, de l'eau, du méthanol, un polyméthylol de formule (I) 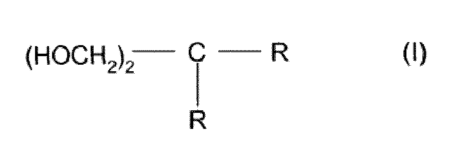 un méthylolalcanal de formule (II) 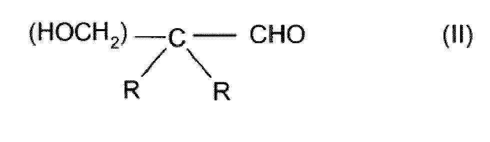 un alcool de formule (III), 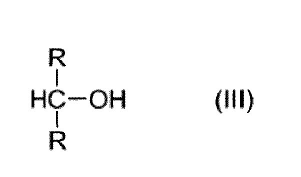 et un alcanal contenant un groupe méthylène en position α par rapport au groupe carbonyle,
les R signifiant à chaque fois indépendamment les uns des autres un groupe méthylol supplémentaire ou un groupe alkyle contenant 1 à 22 atomes C ou un groupe aryle ou aralkyle contenant 6 à 22 atomes C,
selon lequel, lors d'une première étape de distillation, une séparation du mélange de composants de point d'ébullition faible en une fraction principalement riche en eau de point d'ébullition plus élevé et en une fraction organique aqueuse de point d'ébullition plus faible contenant l'amine tertiaire a lieu,
et, lors de la deuxième étape de distillation, la fraction organique aqueuse issue de la première étape de distillation est séparée en une fraction contenant principalement l'amine et en une autre fraction appauvrie en amine,
**caractérisé en ce que** l'amine tertiaire est la triméthylamine ou la triéthylamine et la température de fond lors de la deuxième étape de distillation est de 110 °C et plus.

2. Procédé selon la revendication 1, **caractérisé en ce qu'**une base est ajoutée au mélange de composants de point d'ébullition faible avant l'introduction dans la première étape de distillation.

3. Procédé selon au moins l'une quelconque des revendications 1 à 2, **caractérisé en ce que** le méthylolalcanal de formule (I) est le néopentylglycol, le triméthylolpropane, la pentaérythrite, le triméthyloléthane ou le triméthylolbutane.

4. Procédé selon au moins l'une quelconque des revendications 1 à 3, **caractérisé en ce que** l'amine tertiaire est la triméthylamine.

5. Procédé selon au moins l'une quelconque des revendications 1 à 4, **caractérisé en ce que** le mélange de composants de point d'ébullition faible contient
1 à 20 % en poids de méthanol,
0,01 à 1 % d'aldéhyde non réagi,
0,5 à 5 % en poids d'alcool (III),
0,5 à 5 % en poids de tert.-amine,
0,01 à 1 % en poids de méthylolalcanal (II),
0 à 5 % en poids de composés secondaires organiques,
le reste étant de l'eau.

6. Procédé selon au moins l'une quelconque des revendications 1 à 5, **caractérisé en ce que** la température de fond lors de la deuxième étape de distillation se situe dans la plage allant de 110 à 145 °C.

7. Procédé selon au moins l'une quelconque des revendications 1 à 6, **caractérisé en ce que** le temps de séjour dans la deuxième étape de distillation est dans la plage allant de 5 à 45 minutes.

8. Procédé selon au moins l'une quelconque des revendications 1 à 7, **caractérisé en ce que** la pression lors de la deuxième étape de distillation se situe dans la plage allant de 3 à 10 bar.

9. Procédé selon au moins l'une quelconque des revendications 1 à 8, **caractérisé en ce que** la fraction contenant principalement l'amine est traitée et utilisée en tant que catalyseur pour la réaction d'alcanals avec du formaldéhyde.

10. Procédé selon au moins l'une quelconque des revendications 1 à 9, **caractérisé en ce que** l'amine utilisée est la triéthylamine et la fraction contenant principalement l'amine issue de la deuxième étape de distillation est séparée lors d'une troisième étape de distillation en une fraction qui contient principalement l'amine tertiaire et en une fraction qui contient principalement de l'eau ou du méthanol.

11. Procédé selon au moins l'une quelconque des revendications 1 à 9, **caractérisé en ce que** l'amine utilisée est la triméthylamine et la fraction organique aqueuse issue de la deuxième étape de distillation est séparée en une phase méthanolique et une fraction organique aqueuse.

12. Procédé selon la revendication 10 ou la revendication 11, **caractérisé en ce que** la deuxième et la troisième étape de distillation sont réalisées ensemble dans une colonne à paroi de séparation.

13. Procédé selon la revendication 10 ou la revendication 12, **caractérisé en ce que** la triéthylamine issue de la troisième étape de distillation est utilisée en tant que catalyseur pour la réaction d'alcanals avec du formaldéhyde.

14. Procédé selon la revendication 11 ou la revendication 12, **caractérisé en ce que** la triméthylamine issue de la deuxième étape de distillation est utilisée en tant que catalyseur pour la réaction d'alcanals avec du formaldéhyde.

15. Procédé selon au moins l'une quelconque des revendications 1 à 14, **caractérisé en ce que** le mélange de composants de point d'ébullition faible est obtenu par un procédé d'hydrogénation à plusieurs étapes, selon lequel, à l'étape a), des alcanals sont condensés avec du formaldéhyde en présence d'amines tertiaires en tant que catalyseur pour former des méthylolalcanals de formule (II), puis, à l'étape b), le mélange réactionnel obtenu à l'étape a) est séparé par distillation en un fond, qui contient principalement les composés de formule (II), et un courant de tête, qui contient les composants de point d'ébullition faible, et, à l'étape c), la sortie de fond de l'étape b) est hydrogénée, puis, lors d'une étape d), la sortie de l'étape c) est distillée, le mélange de composants de point d'ébullition faible étant séparé à l'étape d).
